(19) Europäisches Patentamt
European Patent Office
Office européen des brevets

(11) EP 0 432 984 B1

(12) **EUROPEAN PATENT SPECIFICATION**

(45) Date of publication and mention of the grant of the patent:
**04.03.1998 Bulletin 1998/10**

(51) Int. Cl.[6]: **C07D 209/24**, C07C 211/15, C07C 211/07, A61K 31/40

(21) Application number: **90313373.4**

(22) Date of filing: **10.12.1990**

(54) **Carbamoyl derivatives**

Carbamoylderivate

Dérivés de carbamoyle

(84) Designated Contracting States:
**AT BE CH DE DK ES FR GB GR IT LI LU NL SE**

(30) Priority: **11.12.1989 GB 8927981**

(43) Date of publication of application:
**19.06.1991 Bulletin 1991/25**

(73) Proprietor: **ZENECA LIMITED**
**London W1Y 6LN (GB)**

(72) Inventor: **Jacobs, Robert Toms**
**Wilmington, Delaware 19808 (US)**

(74) Representative:
**Smith, Stephen Collyer et al**
**Intellectual Property Department**
**ZENECA Pharmaceuticals**
**Mereside**
**Alderley Park**
**Macclesfield Cheshire SK10 4TG (GB)**

(56) References cited:
**EP-A- 0 114 950** **EP-A- 0 220 066**
**EP-A- 0 290 145**

- **CHEMISCHE BERICHTE vol. 119, no. 7, 4 July 1986, WEINHEIM pages 2233 - 2248; KARL-RUDOLF GASSEN ET AL.: 'Zum Einfluss von Trifluormethylgruppen auf die Reaktionen aliphatischer Diazonium-Ionen und Carbokationen'**
- **J.MED.CHEM (1994), 37, pages 1282-1297**

Remarks:
The file contains technical information submitted after the application was filed and not included in this specification

EP 0 432 984 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Xerox (UK) Business Services
2.15.12/3.4

## Description

This invention concerns a novel carbamoyl derivatives, and, more particularly, a novel 5-(2-methyl-4,4,4-trifluorobutylcarbamoyl)indole derivatives which antagonize the pharmacological actions of one or more of the arachidonic acid metabolites known as leukotrienes (hereafter referred to as "leukotriene antagonist properties"). The novel derivatives are useful whenever such antagonism is desired. Thus, such compounds may be of value in the treatment of those diseases in which leukotrienes are implicated, for example, in the treatment of allergic or inflammatory diseases, or of endotoxic or traumatic shock conditions. The invention also provides pharmaceutical compositions containing the novel derivatives for use in such treatments, methods for their use and processes and intermediates for the manufacture of the novel derivatives.

In European Patent Application publication number 220,066 there is disclosed a series of carbamoyl heterocycles which includes 5-carbamoylindole derivatives of formula Ia (formula set out hereinbelow following the Examples, together with other formulae denoted by Roman numerals) wherein, inter alia, $R^1$ includes (2-10C)alkyl optionally containing 1 or more fluorine substituents, Ra is hydrogen or methyl, Rc is hydrogen or (1-4C)alkoxy, Rd includes hydrogen and (1-10C)alkyl, and M includes a residue of formula $-CO.NH.SO_2R^6$ in which the values of $R^6$ include (6-12C)aryl which may bear 1 or 2 substituents selected from a group consisting of halogeno, amino, (1-4C)alkyl, (1-4C)alkoxy and trifluoromethyl, and the pharmaceutically acceptable salts thereof. It has now been discovered, and herein lies the basis of the claimed invention, that particularly useful leukotriene antagonist properties are shown by novel carbamoyl derivates of formula Ia in which $R^1$ is 2-methyl-4,4,4-trifluorobutyl, and the other groups have specified values, as defined below.

According to the invention there is provided a 5-(2-methyl-4,4,4-trifluorobutylcarbamoyl)indole derivative of formula I both in racemic form and in the form of a substantially pure enantiomer, particularly the (R)-form; or a pharmaceutically acceptable salt thereof.

It will be appreciated that, owing to the asymmetrically substituted carbon atom in the 2-methyl-4,4,4-trifluorobutylcarbamoyl group, the compound of formula I may exist in, and be isolated in, optically-active and racemic forms. The compound may exhibit polymorphism. The compound may form solvates. It is to be understood that the present invention encompasses any racemic, optically-active or polymorphic form, or solvate, or mixtures thereof, which form possesses leukotriene antagonist properties, it being well known in the art how to prepare optically-active forms (for example, by resolution of the racemic form or by synthesis from optically-active starting materials) and how to determine the leukotriene antagonist properties by the standard tests described hereinafter. It may be preferred to use the compound of formula I in a form which is characterized as containing, for example, at least 95%, 98% or 99% enantiomeric excess (ee) of the (R)-form.

It is preferred that the 2-methyl-4,4,4-trifluorobutyl carbamoyl group be of the optically active (R)-form.

Specific forms of the compound of the invention are described in the accompanying examples and may be used either in the free acid form or as a corresponding pharmaceutically acceptable salt.

Examples of suitable pharmaceutically acceptable salts are salts formed with bases which form a physiologically acceptable cation, such as alkali metal (especially lithium, sodium and potassium), alkaline earth metal (especially calcium and magnesium), aluminum and ammonium salts, as well as salts made with appropriate organic bases such as triethylamine, morpholine, piperidine and triethanolamine.

A compound of formula I may be made by processes which include processes well known in the chemical art for the production of structurally analogous carbocyclic compounds. Such processes for the manufacture of a compound of formula I as defined above are provided as further features of the invention and are illustrated by the following procedures in which the meanings of generic radicals are as defined:

(A) Reacting a corresponding compound of formula II wherein T is carboxy (which compound is hereinafter referred to as "benzoic acid of formula II") with 2-methylbenzenesulfonamide in the presence of a dehydrating agent or reacting a reactive derivative of a benzoic acid of formula II with 2-methylbenzenesulfonamide, or a salt thereof.

Thus, for example, a free benzoic acid of formula II may be reacted with a suitable dehydrating agent, for example, with dicyclohexylcarbodiimide or 1-(3-dimethylaminopropyl)-3-ethylcarbodiimide, or with a hydrochloride or hydrobromide salt thereof, optionally together with an organic base, for example, 4-dimethylaminopyridine, and with 2-methylbenzenesulfonamide in the presence of a suitable solvent or diluent, for example, methylene chloride, at a temperature in the range of, for example, 10 to 50 °C, but preferably at or near ambient temperature.

Alternatively, a reactive derivative of a benzoic acid of formula II, for example, an acid halide (such as the acid chloride), acid anhydride or a mixed acid anhydride (such as that formed from N,N-diphenylcarbamic acid and the benzoic acid of formula II by reaction of the sodium salt of the latter acid with N,N-diphenylcarbamoylpyridinium chloride), may be reacted with an alkali metal salt (such as the lithium, sodium or potassium salt) of 2-methylbenzenesulfonamide, conveniently at or near ambient temperature and in a suitable solvent or diluent, for example, tetrahydrofuran, dimethylformamide or methylene chloride.

A benzoic acid of formula II wherein T is a carboxy group may be obtained by decomposing a suitable benzoic ester of formula II in which T is COOR$^h$ wherein R$^h$ is a conveniently removed acid protecting group (which compound is hereinafter referred to as "benzoic ester of formula II"), for example, phenyl, benzyl, or (1-6C)alkyl optionally bearing an acetoxy, (1-4C)alkoxy or (1-4C)alkylthio substituent. A particular value for R$^h$ is, for example, methyl, ethyl, propyl, t-butyl, acetoxymethyl, methoxymethyl, 2-methoxyethyl, methylthiomethyl, phenyl, or benzyl. A preferred value for R$^h$ is methyl.

It will be appreciated that the decomposition of a benzoic ester of formula II can be performed using any one of a variety of procedures well known in the art of organic chemistry. A preferred method for decomposing an ester of formula II comprises reacting the ester with a suitable base, for example, as described in Example 1.f. When such a method is employed, the resulting benzoic acid of formula II, wherein T is a carboxy group, is initially obtained as the corresponding salt of the base used for the hydrolysis and may be isolated as such or converted to the free acid form by a conventional acidification procedure, for example, by reaction with a suitable strong acid such as hydrochloric or sulfuric acid.

(B) Acylating 2-methyl-4,4,4-trifluorobutylamine with a carboxylic acid of formula III wherein U is carboxy (which compound is hereinafter referred to as "indole carboxylic acid of formula III") in the presence of a dehydrating agent or with a reactive derivative of an indole carboxylic acid of formula III. It will be clear to one skilled in the art that use of racemic 2-methyl-4,4,4-trifluorobutylamine will afford a racemic carbamoyl derivative of formula I and that use of 2-methyl-4,4,4-trifluorobutylamine which is substantially enantiomerically pure will afford a corresponding carbamoyl derivative of formula I which is substantially enantiomerically pure.

Thus, for example, an indole carboxylic acid of formula III may be reacted with a suitable dehydrating agent, for example, with 1-(3-dimethylaminopropyl)-3-ethyl carbodiimide, or with a hydrochloride or hydrobromide salt thereof, optionally together with an organic base, for example, 4-dimethylaminopyridine, and with 2-methyl-4,4,4-trifluorobutylamine, or with a salt thereof, especially a hydrochloride or hydrobromide salt, optionally together with an organic base, for example, 4-dimethylaminopyridine, in the presence of a suitable solvent or diluent, for example tetrahydrofuran or 1,2-dimethoxyethane, at a temperature in the range of, for example 10 to 85 °C, for example in tetrahydrofuran at or near 67 °C.

Alternatively, a reactive derivative of an indole acid of formula III, for example, an acid halide (such as the acid chloride), acid anhydride or mixed acid anhydride (such as that formed with ethyl chloroformate in the presence of an organic base such as, for example triethylamine or 4-dimethylaminopyridine) or a lower alkyl ester (such as the methyl ester) may be used as the acylating agent, conveniently together with a suitable inert solvent or diluent, for example dichloromethane, tetrahydrofuran or 1,2-dimethoxyethane.

An indole carboxylic acid of formula III wherein U is a carboxy group may be obtained by decomposing a suitable indole ester of formula III in which U is COOR$^j$ wherein R$^j$ is a conveniently removed acid protecting group (which compound is hereinafter referred to as "indole ester of formula III"), for example, phenyl, benzyl, or (1-6C)alkyl optionally bearing an acetoxy, (1-4C)alkoxy or (1-4C)alkylthio substituent. A particular value for R$^j$ is, for example, methyl, ethyl, propyl, t-butyl, acetoxymethyl, methoxymethyl, 2-methoxyethyl, methylthiomethyl, phenyl, or benzyl. Preferred values for R$^j$ include methyl and benzyl.

It will be appreciated that the decomposition of an indole ester of formula III can be performed using any one of a variety of procedures well known in the art of organic chemistry. A preferred method for decomposing an indole ester of formula III comprises reacting the ester with a suitable base, for example, as described in Example 3.c. When such a method is employed, the resulting indole carboxylic acid of formula III, wherein U is a carboxy group, is initially obtained as the corresponding salt of the base used for the hydrolysis and may be isolated as such or converted to the free acid form by a conventional acidification procedure, for example, by reaction with a suitable strong acid such as hydrochloric or sulfuric acid.

The novel 2-methyl-4,4,4-trifluorobutylamine, both in racemic form and in the form of a substantially pure enantiomer, particularly the (R)-form, (preferably isolated as an acid addition salt, for example the hydrochloride), and its preparation provide further aspects of the invention based upon the utility as a chemical intermediate. The novel amine may be prepared in racemic or optically active form from 4,4,4-trifluorobutyric acid or an ester thereof, for example the ethyl ester, as described in the Examples. Thus, in Example 1, parts g.-j. and parts k.-u., two similar procedures for preparation of racemic 2-methyl-4,4,4-trifluorobutylamine hydrochoride are described. Preparation of the optically active (R)-2-methyl-4,4,4-trifluorobutylamine, isolated as the hydrochloride salt, using the chiral auxiliary reagent (4R,5S-(+)-4-methyl-5-phenyl-2-oxazolidinone, is described in Example 2, parts a.-g.

If a compound of formula I in substantially enantiomerically pure form is required, it may be obtained by a procedure using enantiomerically pure starting materials, as described above, or by separation of the desired optically active form using a conventional method. If a compound of formula I is obtained and a pharmaceutically acceptable salt thereof is required, the pharmaceutically acceptable salt may be obtained by reacting the compound of formula I with a suitable base which affords a physiologically acceptable cation.

The necessary starting materials for the above procedures may be made by procedures which are selected from standard techniques of organic chemistry, techniques which are analogous to the synthesis of known, structurally similar compounds, and techniques which are analogous to the above described methods and those described in the Examples.

The starting materials of formulae II and III conveniently may be prepared beginning with indole-5-carboxylic acid (formula IV wherein U is carboxy). Thus, the acid of formula IV wherein U is carboxy may be esterified by a conventional method to form a corresponding ester of formula IV wherein U has the value $COOR^j$ and $R^j$ is defined as above. An ester of formula IV wherein U is $COOR^j$ may be substituted at the 3-position of the indole using an α-bromo toluic ester of formula V wherein T is $COOR^h$ and $R^h$ has the values defined above, using a similar method to that described in Example 1.b. to provide a corresponding diester of formula VI wherein T is $COOR^h$ and U is $COOR^j$. An α-bromo toluic ester of formula V may be prepared by a conventional method, for example as described in European Patent Application publication number 220,066 or in U.S. patent 4,859,692. A diester of formula VI may be converted into a corresponding diester of formula VII by alkylation at the 1-position of the indole using a similiar procedure to that described in Example 1.c. and a conventional alkylating agent, for example methyl iodide.

By selective conversion of the ester group of formula $COOR^j$ into a carboxy group, a diester of formula VII wherein T is $COOR^h$ and U is $COOR^j$ may be converted into a corresponding indole carboxylic acid of formula VII wherein T is $COOR^h$ and U is carboxy. For example, a diester of formula VII wherein T is $COOR^h$ in which $R^h$ is methyl and U is $COOR^j$ in which $R^j$ is benzyl may be converted into a corresponding indole carboxylic acid of formula VII wherein U is carboxy and T is $COOR^h$ in which $R^h$ is methyl by hydrogenolysis of the benzyl group using a similar method to that described in Example 1.d. The resulting indole carboxylic acid of formula VII may be converted into a corresponding starting material benzoic ester of formula II wherein T is $COOR^h$ using a similiar procedure to that of (B) above, for example as described in Example 1.e. and Example 2.h. Clearly, use of racemic 2-methyl-4,4,4-trifluorobutylamine will afford racemic benzoic ester of formula II and use of substantially enantiomerically pure amine will afford substantially enantiomerically pure benzoic ester of formula II.

Alternatively, by selective conversion of the ester group of formula $COOR^h$ into a carboxy group, a diester of formula VII wherein T is $COOR^h$ and U is $COOR^j$ may be converted into a corresponding benzoic acid of formula VII wherein T is carboxy and U is $COOR^j$. For example a dimethyl ester of formula VII wherein T is $COOR^h$ and U is $COOR^j$ in which both $R^h$ and $R^j$ are methyl may be hydrolyzed selectively by a similar method to that described in Example 3.a. to afford a benzoic acid of formula VII wherein T is carboxy and U is $COOR^j$ in which $R^j$ is methyl. The resulting benzoic acid of formula VII may be converted into a starting material indole ester of formula III wherein U is $COOR^j$ using a similar procedure to (A) above, for example as described in Example 3.b.

The starting materials of formula II wherein T is carboxy or $COOR^h$, both in racemic form and in substantially enantiomerically pure form, are novel and are provided as further features of the invention based upon their utility as chemical intermediates. When a starting material of formula II which is substantially enantiomerically pure is required, it will be clear to one skilled in the art that the starting material may be prepared using substantially enantiomerically pure 2-methyl-4,4,4-trifluorobutylamine or that the racemic starting material may be resolved by a conventional method.

As stated previously, the compound of formula I possesses leukotriene antagonist properties. Thus, it antagonises at least one of the actions of one or more of the arachidonic acid metabolites known as leukotrienes, for example, $C_4$, $D_4$, and/or $E_4$, which are known to be powerful spasmogens (particularly in the lung), to increase vascular permeability and to be implicated in the pathogenesis of asthma and inflammation, as well as of endotoxic shock and traumatic shock. The compound of formula I is thus useful in treatment of diseases in which leukotrienes are implicated and in which antagonism of their action is desired. Such diseases include, for example, allergic pulmonary disorders such as asthma, hay fever and allergic rhinitis and certain inflammatory diseases such as bronchitis, ectopic and atopic eczema, and psoriasis, as well as vasospastic cardiovascular disease, and endotoxic and traumatic shock conditions.

The compound of formula I is a potent leukotriene antagonist and is useful whenever such activity is desired. For example, the compound of formula I is of value as a pharmacological standard for the development and standardization of new disease models and assays for use in developing new therapeutic agents for treating the diseases in which the leukotrienes are implicated.

When used in the treatment of one or more of the above mentioned diseases, the compound of formula I is generally administered as an appropriate pharmaceutical composition which comprises the compound of formula I as defined hereinbefore together with a pharmaceutically acceptable diluent or carrier, the composition being adapted for the particular route of administration chosen. Such compositions are provided as a further feature of the invention. They may be obtained employing conventional procedures and excipients and binders and may be in a variety of dosage forms. For example, they may be in the form of tablets, capsules, solutions or suspensions for oral administration; in the form of suppositories for rectal administration; in the form of sterile solutions or suspensions for administration by intravenous or intramuscular injection or infusion; in the form of aerosols or nebuliser solutions or suspensions for administration by inhalation; and in the form of powders together with pharmaceutically acceptable inert solid diluents such as lactose for administration by insufflation. If a solid form of a compound of formula I is required, it may be preferred to use an amorphous form, which amorphous form may be prepared by adding an aqueous acid, for example hydrochloric acid, to a solution of the sodium salt of the compound of formula I in an alcohol-water mixture, for example methanol-water mixture, to precipitate the compound of formula I.

For oral administration a tablet or capsule containing up to 250 mg (and typically 5 to 100 mg) of the compound of formula I may conveniently be used. Similarly, for intravenous or intramuscular injection or infusion a sterile solution or suspension containing up to 10% w/w (and typically 0.05 to 5% w/w) of the compound of formula I may conveniently be used.

The dose of the compound of formula I to be administered will necessarily be varied according to principles well known in the art taking account of the route of administration and the severity of the condition and the size and age of the patient under treatment. However, in general, the compound of formula I will be administered to a warm-blooded animal (such as man) so that a dose in the range of, for example, 0.01 to 25 mg/kg (and usually 0.1 to 5 mg/kg) is received.

The leukotriene antagonist properties of the compound of formula I may be demonstrated using standard tests. Thus, for example, they may be demonstrated in vitro using the standard guinea-pig tracheal strip preparation described by Krell (J. Pharmacol. Exp. Ther., 1979, 211, 436) and as also described in European Patent Application publication number 220,066 and in U.S. patent 4,859,692.

The selectivity of action of compounds as leukotriene antagonists as opposed to non-specific smooth muscle depressants may be shown by carrying out the above in vitro procedure using the non-specific spasmogen barium chloride at a concentration of $1.5 \times 10^{-3}$M, again in the presence of indomethacin at $5 \times 10^{-6}$M.

Alternatively, the antagonistic properties of the compound of formula I can be demonstrated in vitro by a receptor-ligand binding assay described by Aharony (Fed. Proc., 1987, 46, 691). According to this procedure, membrane fractions, containing the $LTD_4/E_4$ receptors, are prepared from guinea-pig lung parenchyma and incubated for 30 minutes at 22 °C with 1nM $^3H$-$LTD_4$ in the absence or presence of tested antagonist. Specific binding, determined under conditions that prevent enzymatic metabolism of $^3H$-$LTD_4$, is the net result of total $^3H$-$LTD_4$ binding minus nonspecific binding determined in the presence of 1-2000 fold excess unlabelled $LTD_4$. Each assay is done in duplicate and results (Ki values) are typically a mean of several such determinations in individual receptor batches.

The % inhibition by a tested antagonist, relative to control binding (vehicle alone), is expressed as a fraction of log[antagonist] concentration (in molar units) and the half-maximal inhibition ($IC_{50}$) determined by computerized non-linear least-square analysis. The binding constant (Ki) is then calculated from $IC_{50}$ by the Cheng-Prusoff equation:

$$Ki= \frac{IC_{50}}{\left[1 + \frac{[L]}{Kd}\right]}$$

where [L] is $^3H$-$LTD_4$ concentration and Kd is the affinity constant of $LTD_4$ to this receptor, determined separately for each batch. (Biochem. Pharmacol., 1973, 22, 3099-3108).

In general, the compounds of formula I tested demonstrated statistically significant activity as $LTC_4$, $LTD_4$ and/or $LTE_4$ antagonists in one of the above tests at a concentration of about $10^{-8}$M or much less. For example, a pKi value of 9.4 was typically determined for the compound of Example 2.

Activity as a leukotriene antagonist may also be demonstrated in vivo in laboratory animals, for example, in a routine guinea-pig aerosol test described by Snyder, et al. (J. Pharmacol. Methods, 1988, 19, 219). In this test the particularly useful leukotriene antagonist properties of the carbamoyl derivative of formula I may be demonstrated. According to this procedure, guinea-pigs are pre-dosed with test compound as a solution in poly(ethylene glycol) (generally 1 hour) before an aerosol challenge of leukotriene $LTD_4$ (starting with 2 ml of a 30 microgram/ml solution) and the effect of the test compound on the average time of leukotriene initiated change in breathing pattern (such as onset of dyspnea) recorded and compared with that in undosed, control guinea-pigs. Percent protection engendered by a test compound was calculated from the time delay to the onset of dyspnea compared to that for control animals. Typically, an $ED_{50}$ of 1.1 µmol/kg for the compound of Example 2 following oral administration was determined, without any indication of untoward side-effects at several multiples of the minimum effective dose. By way of comparison, an oral $ED_{50}$ of 19.2 µmol/kg was measured for the compound of formula Ia wherein $R^1$ is cyclopentylmethyl, Ra is hydrogen, Rd is methyl, Rc is methoxy, and M is a residue of formula -CO.NH.$SO_2$ $R^6$ in which $R^6$ is 2-methylphenyl (Example 10 of European Patent Application publication number 220,066).

The invention will now be illustrated by the following non-limiting examples in which, generally, unless stated otherwise:

(i) temperatures are given in degrees Celsius (°C); operations were carried out at room or ambient temperature, that is, at a temperature in the range of 18-25 °C; air or moisture sensitive reactions were performed under an inert

(argon or nitrogen) atmosphere;

(ii) evaporation of solvent was carried out using a rotary evaporator under reduced pressure (600-4000 pascals; 4.5-30 mm Hg) with a bath temperature of up to 60 °C;

(iii) flash chromatography was carried out on Merck Kieselgel (Art 9385) and column chromatography on Merck Kieselgel 60 (Art 7734); [these materials were obtained from E. Merck, Darmstadt, W. Germany]; thin layer chromatography (TLC) was carried out on Analtech 0.25 mm silica gel GHLF plates (Art 21521), obtainable from Analtech, Newark, DE, USA; gas-liquid chromatography (GLC) was carried out on a 0.2 mm x 25 m fused silica glass capillary column with 5% phenyl methyl silicone as the stationary phase, with a flow rate of 0.7 mL/min and an oven temperature program of 50 °C for 5 min, then 10 °C/min increase to 275 °C; the injector temperature was 225 °C and detector 275 °C; retention times ($t_R$) are given in min;

(iv) in general, the course of reactions was followed by TLC and reaction times are given for illustration only;

(v) melting points are uncorrected and (d) indicates decomposition; the melting points given are those obtained for the materials prepared as described; polymorphism may result in isolation of materials with different melting points in some preparations;

(vi) all final products were essentially pure by TLC and had satisfactory nuclear magnetic resonance (NMR) spectra and microanalytical data;

(vii) yields are given for illustration only;

(viii) when given, NMR data is in the form of delta values for major diagnostic protons, given in parts per million (ppm) relative to tetramethylsilane (TMS) as an internal standard, determined at 80 MHz, 250 MHz, 300 MHz or 400 MHz using $CDCl_3$, DMSO-$d_6$ or $CD_3OD$ as solvent; conventional abbreviations for signal shape are used, for example: s, singlet; d, doublet; m, multiplet; br, broad; etc.; the "observed" (rather than calculated) shifts for complex signals are reported; in addition "Ar" signifies an aromatic group or signal; measurements of enantiomeric excess (ee) were made by $^{19}F$ NMR using the chiral shift reagent, 2,2,2-trifluoro-1-(9-anthryl)ethanol-$d_{11}$ (TFAE-$d_{11}$); the fluorine resonance for a compound of formula I dissolved in $CDCl_3$ which appears at about -63.8 ppm from $CFCl_3$ when measured at 376.5 MHz demonstrates a greater shift of the signal for the (R)-isomer than for the signal of the (S)-isomer in the presence of added (R)-(-)-TFAE-$d_{11}$;

(ix) reduced pressures are given as absolute pressures in pascals (Pa); other pressures are given as gauge pressures in bars;

(x) chemical symbols have their usual meanings; in general the symbols for units of the SI system or those accepted for use with the SI system are used (e.g., L, mL, g, mg, h, min); the following abbreviations have also been used: v (volume), w (weight), mp (melting point), bp (boiling point);

(xi) solvent ratios are given in volume: volume (v/v) terms; and

(xii) mass spectra (MS) were run with an electron energy of 70 electron volts in the chemical ionization (CI) mode or electron impact (EI) mode; generally only the peak attributable to the parent ion is reported.

## Example 1

3-Methoxy-4-[1-methyl-5-(2-methyl-4,4,4-trifluorobutylcarbamoyl)indol-3-ylmethyl]-N-(2-methylphenylsulfonyl)benzamide.

A solution of 3-methoxy-4-[1-methyl-5-(2-methyl-4,4,4-trifluorobutylcarbamoyl)indol-3-ylmethyl]benzoic acid (250 mg), 4-dimethylaminopyridine (69.8 mg), 1-(3-dimethylaminopropyl)-3-ethylcarbodiimide hydrochloride (127 mg) and 2-methylbenzenesulfonamide (95.4 mg) in methylene chloride (5 mL) was stirred under a nitrogen atmosphere for 24 hours. The mixture was diluted with methylene chloride, washed (10% (w/v) hydrochloric acid, water), and evaporated. The resulting rose-colored foam was dissolved in methylene chloride (5 mL), filtered through a 0.45 micron membrane filter, and precipitated by addition to hexane (50 mL). The solid was collected by filtration to give the title compound (189.2 mg, 57%) as a pale pink powder; mp 147-149 °C.

| Analysis for $C_{31}H_{32}F_3N_3O_5S$: | | | |
|---|---|---|---|
| Calculated: | C, 60.48; | H, 5.24; | N, 6.83 |
| Found: | C, 60.39; | H, 5.60; | N, 6.59 |

The starting benzoic acid was prepared as follows:

a. Benzyl indole-5-carboxylate.

A solution of indole-5-carboxylic acid (68.3 g), benzyl alcohol (64.9 g) and triphenylphosphine (157.0 g) in tetrahydrofuran (1.2 L) was cooled to 5 °C and treated dropwise with diethyl azodicarboxylate (90.0 g). Upon completion of the addition, the mixture was allowed to warm to room temperature. The reaction was stirred for 24 hours and then evaporated. The residue was taken up in diethyl ether (1 L) and filtered. The filtrate was evaporated to give a yellow syrup which was purified by flash chromatography, eluting sequentially with 2:1, 1:1 and 1:2 hexane:methylene chloride, to afford a yellow-white solid. This material was triturated with 1:1 hexane: methylene chloride (300 mL) and filtered to give benzyl indole-5-carboxylate as a white solid (74.2 g, 70%); mp 127-129 °C; partial NMR (300 MHz, $CDCl_3$): 5.39 (s, 2H, $CH_2$), 6.61 (m, 1H, indole-H(2)), 8.56 (br, 1H, NH).

b. Methyl 4-(5-benzyloxycarbonylindol-3-ylmethyl)-3-methoxybenzoate.

A solution of benzyl indole-5-carboxylate (86.8 g), methyl 4-bromomethyl-3-methoxybenzoate (89.5 g) and potassium iodide (57.4 g) in N,N-dimethylformamide (900 mL) was heated to 80 °C for 10 hours. The reaction mixture was evaporated and partitioned between diethyl ether and water. The organic layer was separated and washed with water. The aqueous washes were combined and extracted with diethyl ether. The combined organic extract was dried ($MgSO_4$) and evaporated. The residue was purified by flash chromatography, eluting sequentially with 0:1:1, 2:48:50, 4:46:50, 5:45:50, and 10:40:50 ethyl acetate:hexane:methylene chloride, to afford methyl 4-iodomethyl-3-methoxybenzoate (27.8 g), recovered benzyl indole-5-carboxylate (29.6 g), and the crude product as a tan solid (50.6 g). Treatment of the recovered benzyl indole-5-carboxylate (29.6 g) in N,N-dimethylformamide (250 mL) with methyl 4-iodomethyl-3-methoxybenzoate (29.8 g) at 80 °C for 12 hours, followed by evaporation, gave a dark residue, which was dissolved in diethyl ether and washed with water (3 times). The aqueous washes were combined and extracted with diethyl ether. The combined organic extract was dried ($MgSO_4$) and evaporated. The residue was purified by flash chromatography, eluting sequentially with 0:1:1, 2:48:50, 5:45:50, and 10:40:50 ethyl acetate:hexane:methylene chloride, to give further crude product as a tan solid (31.9 g). The combined crude product (82.5 g) was suspended in diethyl ether (400 mL), heated to reflux for 30 min, cooled and filtered to obtain methyl 4-(5-benzyloxycarbonylindol-3-ylmethyl)-3-methoxybenzoate as an ivory solid (46.1 g, 31%); partial NMR (250 MHz, $CDCl_3$): 3.84 (s, 3H, $CO_2CH_3$), 3.88 (s, 3H, $OCH_3$), 4.14 (s, 2H, $CH_2$), 5.35 (s, 2H, $OCH_2$), 6.97 (d, 1H, indole-H(2)), 8.15 (br, 1H, NH), 8.37 (s, 1H, indole-H(4)).

c. Methyl 4-(5-benzyloxycarbonyl-1-methylindol-3-ylmethyl)-3-methoxybenzoate.

A solution of methyl 4-(5-benzyloxycarbonylindol-3-ylmethyl)-3-methoxybenzoate (46.1 g) in N,N-dimethylformamide (200 mL) was added to a slurry of sodium hydride (2.83 g) in N,N-dimethylformamide (300 mL) at 5 °C under a nitrogen atmosphere. The mixture was stirred for 30 minutes at 5 °C, then was treated with iodomethane (16.6 g), allowed to warm to room temperature and stirred for 16 hours. The reaction mixture was then poured into ice/water (400 mL), diluted with water (250 mL) and 1N hydrochloric acid (250 mL). The resulting aqueous solution was extracted with ethyl acetate. The combined organic extract was washed (1N hydrochloric acid, water, brine), dried ($MgSO_4$), filtered and evaporated. The residue was triturated with warm diethyl ether and filtered to give methyl 4-(5-benzyloxycarbonyl-1-methylindol-3-ylmethyl)-3-methoxybenzoate as an ivory solid (42.4 g, 89%); partial NMR (300 MHz, $CDCl_3$): 3.75 (s, 3H, $NCH_3$), 3.87 (s, 3H, $CO_2CH_3$), 3.90 (s, 3H, $OCH_3$), 4.12 (s, 2H, $CH_2$), 5.36 (s, 2H, $OCH_2$), 6.82 (s, 1H, indole-H(2)), 8.38 (d, 1H, indole-H(4)).

d. Methyl 4-(5-carboxy-1-methylindol-3-ylmethyl)-3-methoxybenzoate.

A solution of methyl 4-(5-benzyloxycarbonyl-1-methylindol-3-ylmethyl)-3-methoxybenzoate (41.0 g) and formic acid (40 mL) in N,N-dimethylformamide (600 mL) was treated with 10% (w/w) palladium on carbon (10 g) and shaken under hydrogen (3.45 bar) for 24 hours. The catalyst was removed by filtration through diatomaceous earth and the filtrate evaporated to give an amber solid. The solid was triturated with warm diethyl ether and filtered to afford methyl 4-(5-carboxy-1-methylindol-3-ylmethyl)-3-methoxybenzoate as a light gray solid (28.9 g, 88%); mp 249-251 °C; partial NMR (250 MHz, DMSO-$d_6$): 3.78 (s, 3H, $NCH_3$), 3.64 (s, 3H, $CO_2CH_3$), 3.93 (s, 3H, $OCH_3$), 4.09 (s, 2H, $CH_2$), 7.12 (s, 1H, indole-H(2)), 8.16 (s, 1H, indole-H(4)), 12.44 (br, 1H, $CO_2H$).

e. Methyl 3-methoxy-4-[1-methyl-5-(2-methyl-4,4,4-trifluorobutylcarbamoyl)indol-3-ylmethyl]benzoate.

A solution of methyl 4-(5-carboxy-1-methylindol-3-ylmethyl)-3-methoxybenzoate (2.0 g), 4-dimethylaminopyridine (0.71 g), 1-(3-dimethylaminopropyl)-3-ethylcarbodiimide hydrochloride (1.3 g), triethylamine (1.0 mL) and 4,4,4-trifluoro-2-methylbutylamine hydrochloride (1.2 g) in methylene chloride (28 mL) was stirred under a nitrogen atmosphere

for 18 hours. The mixture was diluted with methylene chloride, washed (10% (w/v) hydrochloric acid, water and brine), dried ($MgSO_4$), and evaporated. The resulting ivory foam was purified by flash chromatography, eluting with 1:1 ethyl acetate:hexane to afford methyl 3-methoxy-4-[1-methyl-5-(2-methyl-4,4,4-trifluorobutylcarbamoyl)indol-3-ylmethyl]benzoate as a white powder (2.2g, 82%); mp 168-170 °C; partial NMR (300 MHz, $CDCl_3$): 1.12 (d, 3H, CHC$\underline{H}_3$), 2.00 (m, 1H), 2.22 (m, 2H), 3.34-3.52 (m, 2H, $NCH_2$), 3.75 (s, 3H, $NCH_3$), 3.90 (s, 3H, $CO_2CH_3$), 3.93 (s, 3H, $OCH_3$), 4.13 (s, 2H, $CH_2$), 6.21 (t, 1H, NH), 6.82 (s, 1H, indole-H(2)), 8.02 (s, 1H, indole-H(4)).

f. 3-Methoxy-4-[1-methyl-5(2-methyl-4,4,4-trifluorobutylcarbamoyl)indol-3-ylmethyl]benzoic acid.

A solution of methyl 3-methoxy-4-[1-methyl-5-(2-methyl-4,4,4-trifluorobutylcarbamoyl)indol-3-ylmethyl]benzoate (0.64 g) in methanol (3.5 mL), tetrahydrofuran (3.5 mL) and water (1.3 mL) was treated with lithium hydroxide monohydrate (0.34 g). The mixture was stirred for 18 hours and the organic solvents evaporated. The resulting aqueous solution was acidified with 10% (w/v) hydrochloric acid. The white precipitate which formed was collected by filtration, washed with water and dried under vacuum to give 3-methoxy-4-[1-methyl-5-(2-methyl-4,4,4-trifluorobutylcarbamoyl)indol-3-ylmethyl]benzoic acid as a white powder (0.55 g, 88%); partial NMR (300 MHz, DMSO-$d_6$): 1.00 (d, 3H, CHC$\underline{H}_3$) 3.21 (m, 2H, $NCH_2$), 3.76 (s, 3H, $NCH_3$), 3.91 (s, 3H, $OCH_3$), 4.07 (s, 2H, $CH_2$), 7.15 (m, 2H, ArH), 7.46 (m, 3H, ArH), 7.68 (dd, 1H, ArH), 8.10 (d, 1H, ArH), 8.44 (t, 1H, NHCO).

The 4,4,4-trifluoro-2-methylbutylamine used in step e., above, was prepared as follows:

g. Ethyl 4,4,4-trifluoro-2-methylbutyrate

A solution of diisopropylamine (19.5 mL) in tetrahydrofuran (200 mL) at 0 °C was treated with n-butyllithium (71 mL, 1.5M in hexanes). The resulting solution was stirred for 30 minutes at 0 °C, then was cooled to -70 °C. A solution of ethyl 4,4,4-trifluorobutyrate (14 mL) in tetrahydrofuran (150 mL) was slowly added to the lithium diisopropylamide solution and the resulting mixture was stirred at -70 °C for 30 minutes. A solution of iodomethane (11.5 mL) in tetrahydrofuran was added in one portion, the cooling bath was removed, and the reaction mixture was allowed to warm to room temperature. The reaction mixture was quenched with water and evaporated. The residue was dissolved in methylene chloride, washed (10% (w/v) hydrochloric acid, water, and brine), dried ($MgSO_4$), filtered, and evaporated. The resulting pale yellow liquid was purified by distillation to yield ethyl 4,4,4-trifluoro-2-methylbutyrate as a colorless liquid (7.8 g, 46%); bp 125-128 °C; partial NMR: (300 MHz, $CDCl_3$): 1.30 (m, 6H, C$\underline{H}_3$), $CH_2$C$\underline{H}_3$), 2.15 (m, 1H, H-C(3)), 2.64 (m, 1H, H-C(3)), 2.72 (m, 1H, H-C(2)), 4.16 (q, 2H, $OCH_2$).

h. 4,4,4-Trifluoro-2-methylbutyric acid.

A solution of ethyl 4,4,4-trifluoro-2-methylbutyrate (7.7 g) in methanol (21 mL), tetrahydrofuran (21 mL) and water (8.4 mL) was treated with lithium hydroxide monohydrate (3.5 g). The mixture was stirred for 48 hours and the organic solvents evaporated. The resulting aqueous solution was diluted with water and acidified with 6N hydrochloric acid. The aqueous solution was exhaustively extracted with ethyl acetate. The combined organic extract was washed (water and brine), dried ($MgSO_4$), filtered and evaporated to give 4,4,4-trifluoro-2-methylbutyric acid as a pale yellow liquid (6.5 g, 99%); partial NMR (300 MHz, $CDCl_3$): 1.34 (d, 3H, $CH_3$), 2.18 (m, 1H, H-C(3)), 2.67, (m, 1H, H-C(3)), 2.74 (m, 1H, H-C(2)), 10.6 (br, 1H, $CO_2H$).

i. 4,4,4-Trifluoro-2-methylbutyramide.

A solution of 4,4,4-trifluoro-2-methylbutyric acid (6.5 g) in methylene chloride (42 mL) was added to a solution of 1,1-carbonyldiimidazole (7.5 g) in methylene chloride (40 mL). After gas evolution had subsided, the mixture was heated to reflux temperature for 30 minutes. The mixture was cooled to room temperature and anhydrous ammonia was bubbled through the mixture for 20 minutes. The reaction mixture was stirred for 18 hours at room temperature, then was diluted with ethyl acetate, washed (10% (v/v) hydrochloric acid, water and brine), dried ($MgSO_4$), filtered and evaporated. The solid residue was purified by recrystallization from diethyl ether:hexane to give 4,4,4-trifluoro-2-methylbutyramide as a white solid (4.4 g, 69%); mp 90.5-91.5 °C; partial NMR (300 MHz, $CDCl_3$): 1.30 (d, 3H, $CH_3$), 2.13 (m, 1H, H-C(3)), 2.62 (m, 1H, H-C(3)), 2.71 (m, 1H, H-C(2)), 5.56 (br, 2H, $CONH_2$).

j. 4,4,4-Trifluoro-2-methylbutylamine hydrochloride.

A solution of 4,4,4-trifluoro-2-methylbutyramide (3.3 g) in diethyl ether (50 mL) was added to a refluxing slurry of lithium aluminum hydride (1.2 g) in diethyl ether (50 mL) at such a rate to maintain reflux. The reaction was heated at reflux temperature for 2 hours, cooled to 0 °C, and quenched by sequential addition of water (1.2 mL), 10% (w/v) aqueous sodium hydroxide solution (1.2 mL), and water (3.6 mL). The resulting suspension was filtered. The filtrate was dried ($MgSO_4$) and filtered. Anhydrous hydrogen chloride was bubbled through the filtrate for 5 minutes and the solvent evaporated to afford 4,4,4-trifluoro-2-methylbutylamine hydrochloride as a white solid (3.3 g, 88%); mp 224-225 °C; partial NMR (300 MHz, DMSO-$d_6$): 1.04 (d, 3H, $CH_3$), 2.81-2.60 (br, 2H, $NCH_2$), 8.29 (br, 2H, $NH_2$).

An alternative preparation of the amine hydrochloride used in step e., above, is as follows:

k. 2-Methyl-4,4,4-trifluorobutyric acid.

To sodium hexamethyldisilazane (0.945 M in tetrahydrofuran) (667 mL, 0.63 mol) in tetrahydrofuran (0.9 L) at -78 °C, under nitrogen, was added a solution of ethyl 4,4,4-trifluorobutyrate (90.6 mL) in tetrahydrofuran (100 mL). After stirring for 1.5 hour, to the vigorously stirred mixture was added methyl iodide (112 mL) as fast as possible. The reaction was warmed with a 0 °C bath for 2 hours. Methanol (1 L) and 1 N lithium hydroxide (1.2 L) were added and stirring continued for 48 hours. The mixture was acidified with 2N hydrochloric acid and extracted with ethyl acetate. The combined organic phase was washed (brine), dried ($MgSO_4$), and evaporated at 30 °C. After combination with product from separate conversions of ethyl 4,4,4-trifluorobutyrate (97.79 g), distillation afforded 2-methyl-4,4,4-trifluorobutyric acid (173.24 g, 96%) as a brown solid-liquid contaminated with 4,4,4-trifluorobutyric acid and 2,2-dimethyl-4,4,4-trifluorobutyric acid; bp 48.0-108 °C (9,900 Pa); GLC: $t_R$ = 6.1 min.

l. 2-Methyl-4,4,4-trifluorobutyryl chloride.

To 2-methyl-4,4,4-trifluorobutyric acid (172 g) in methylene chloride (150 mL) and N,N-dimethylformamide (3.5 mL) at 0 °C, under nitrogen was added (dropwise) oxalyl chloride (125 mL). The mixture was allowed to warm to ambient temperature and stirred for 16 hours. After distillation of the solvent, distillation using a concentric tube distillation column (40 cm X 15 mm) afforded 2-methyl-4,4,4-trifluorobutyryl chloride (about 99% purity, 79.87 g, 42%); bp 115.0-116.0 °C (at atmospheric pressure, measured at room temperature as 763.27 mm Hg); GLC: $t_R$ = 5.04 min; MS(CI): 139 (M+H-HCl).

m. 2-Methyl-4,4,4-trifluorobutyramide.

Into 2-methyl-4,4,4-trifluorobutyryl chloride (35 g) in methylene chloride (300 mL) at 0 °C, under nitrogen, was bubbled ammonia for 15 min. The mixture was stirred for 1 hour at 0 °C and then at ambient temperature for for 16 hours before ethyl acetate (600 mL) and 1:1 v/v 10% hydrochloric acid:brine (500 mL) were added. After separating the organic layer, the aqueous layer was basified with 1N sodium hydroxide and extracted with ethyl acetate. The organic extract was dried ($MgSO_4$) and evaporated. The residue was combined with the product of an identical reaction. The combined solid was dissolved in ethyl acetate (200 mL) and added to hexane (2 L) to afford 2-methyl-4,4,4-trifluorobutyramide (56.9 g, 91%) as a colorless solid; mp 90.5-91.5 °C; GLC: $t_R$ = 12.04 min, MS(CI): 156 (M+H).

| Analysis for $C_5H_8F_3NO$: | | | |
|---|---|---|---|
| Calculated: | C, 38.72; | H, 5.20; | N, 9.03 |
| Found: | C, 38.59; | H, 5.11; | N, 8.56 |

n. 2-Methyl-4,4,4-trifluorobutylamine hydrochloride.

To a suspension of lithium aluminum hydride (15.5 g) in diethyl ether (290 ml) was added a solution of 2-methyl-4,4,4-trifluorobutyramide (31.74 g) in diethyl ether (0.5 L) at a rate to obtain a gentle reflux. After heating at reflux for 12 hours and cooling to 0 °C, the reaction was quenched with saturated sodium sulfate solution and allowed to warm to ambient temperature. The mixture was dried ($Na_2SO_4$) and filtered through diatomaceous earth with diethyl ether wash. The filtrate was treated with gaseous hydrochloric acid (14.9 g, 0.409 mol) and then the solvent was evaporated. The residue was dissolved in methylene chloride and combined with product from a similar reaction of 2-methyl-4,4,4-trifluorobutyramide (25 g). Recrystallization from methylene chloride and diethyl ether, followed by trituration with ethyl acetate, afforded 2-methyl-4,4,4-trifluorobutylamine hydrochloride (51.35 g, 79%) as a light pink solid; mp 224.5-225.5 °C; MS(CI): 142 (M+H-HCl).

| Analysis for $C_5H_{11}ClF_3N$: | | | |
|---|---|---|---|
| Calculated: | C, 33.81; | H, 6.24; | N, 7.89 |
| Found: | C, 33.93; | H, 6.13; | N, 8.17 |

Example 2

(R)-3-Methoxy-4-[1-methyl-5-(2-methyl-4,4,4-trifluorobutylcarbamoyl)indol-3-ylmethyl]-N-(2-methylphenylsulfonyl)benzamide.

A mixture of (R)-3-methoxy-4-[1-methyl-5-(2-methyl-4,4,4-trifluorobutylcarbamoyl)indol-3-ylmethyl]benzoic acid (14.28 g), 4-dimethylaminopyridine (4.39 g), 1-(3-dimethylaminopropyl)-3-ethylcarbodiimide hydrochloride (8.34 g) and 2-methylbenzenesulfonamide (5.85 g) was dissolved in dry methylene chloride (270 mL) and the solution was stirred under an inert atmosphere for 48 h. The mixture was diluted with methylene chloride (300 mL) and washed three times with 1N hydrochloric acid. The combined hydrochloric washes were back washed with methylene chloride. The combined organic extracts were washed twice with water and evaporated. The residue was dissolved in a small volume of methanol and 1N sodium hydroxide and this solution was purified by flash chromatography over octadecylsilyl bonded silica gel ("Regis PREP-40-ODS" - irregularly shaped 32-74 µ diameter particles, 72% silanol coverage, 21% carbon load) (450 g) eluting with 50:50 methanol:water, pH 7.1. The appropriate fractions (TLC, $R_f$ = 0.73, octadecylisilyl bonded silica gel, 200 µ layer, 12% carbon load, 80:20 methanol water, pH 6.1, 0.1% ammonium acetate buffer) were combined, the methanol evaporated, and the residual aqueous solution acidified to pH 1 with 1N hydrochloric acid. The resultant precipitate was filtered, washed (water), and dried under vacuum to give the title compound (16.7 g, 88%) as a white solid; mp 117-120 °C; ee at least 99%.

| Analysis for $C_{31}H_{32}F_3N_3O_5S$: | | | |
|---|---|---|---|
| Calculated: | C, 60.48; | H, 5.24; | N, 6.83 |
| Found: | C, 60.32; | H, 5.32; | N, 6.66 |

The starting benzoic acid was prepared as follows:

a. 4,4,4-Trifluorobutyric acid.

A solution of lithium hydroxide monohydrate (324 g) in water (1.8 L) was added to a stirred solution of ethyl 4,4,4-trifluorobutyrate (436 g) in methanol (2.0 L) and dry tetrahydrofuran (2.0 L) and the suspension was stirred overnight. After the suspension was partially evaporated, the residue was diluted with water and washed with diethyl ether. The aqueous layer was acidified with 6M hydrochloric acid and extracted with diethyl ether. The combined extracts were washed (brine) dried (MgSO4), and filtered. The filtrate was evaporated and the residue distilled (bp 165-168 °C) to give 4,4,4-trifluorobutyric acid (347 g, 95%); mp 27-30 °C; partial NMR; (300 MHz, $CDCl_3$): 2.33-2.57 (m, 2H, $CF_3C\underline{H_2}$), 2.66 (t, 2H, $C\underline{H_2}CO_2H$).

b. 4,4,4-Trifluorobutyryl chloride.

Dimethyl formamide (1.0 mL) and oxalylchloride (239 mL) were added to a 0 °C solution of 4,4,4-trifluorobutyric acid (343 g) in dry methylene chloride (230 mL) and warmed to room temperature overnight. The methylene chloride was removed by distillation and the residue distilled to yield 4,4,4-trifluorobutyryl chloride (328 g, 85%); bp 103-106 °C; partial NMR (300 MHz, $CDCl_3$): 2.47-2.64 (m, 2H, $CF_3C\underline{H_2}$) 3.19 (t, H, $CH_2COCl$).

c. (4R,5S)-4-Methyl-3-(4,4,4-trifluorobutyryl)-5-phenyl-2-oxazolidinone.

A solution of n-butyllithium (2.0 mole) in hexane was added to a stirred solution of (4R,5S)-(+)-4-methyl-5-phenyl-2-oxazolidinone (353 g) in dry tetrahydrofuran (2500 mL) at -78 °C under an inert atmosphere. The solution was stirred at -70 °C for 15 min, then 4,4,4-trifluorobutyryl chloride (320 g) was added over 30 min at -60 °C and the mixture

warmed to room temperature and stirred overnight. The mixture was evaporated and the residue was partitioned between diethyl ether and water. The ethereal layer was washed (1N hydrochloric acid, brine (twice)), dried ($MgSO_4$), and evaporated to yield crude product (604 g, about 100%). Filtration through 3000 mL of silica gel using 1:1 methylene chloride:hexanes as the eluent afforded a white solid. Recrystallization from methylene chloride:hexanes afforded (4R,5S)-4-methyl-3-(4,4,4-trifluorobutyryl)-5-phenyl-2-oxazolidinone (519 g, 86%); mp 93-95 °C; partial NMR (300 MHz, $CDCl_3$): 0.91 (d, 3H, $CH_3$), 2.45-2.65 (m, 2H, $CF_3CH_2$), 3.18-3.40 (m, 2H, $CH_2CO$), 4.78 (m, 1H, 4-H oxazolidinone), 5.70 (d, 1H, 5-H oxazolidone), 7.30-7.44 (m, 5H, Ar).

d. (4R,5S)-4-Methyl-3-((2R)-2-methyl-4,4,4-trifluorobutyryl)-5-phenyl-2-oxazolidinone.

To a stirred solution of sodium bis(trimethylsilylamide) (1.9 mole) in tetrahydrofuran (1900 mL) cooled to -40 °C was added a solution of (4R,5S)-4-methyl-3-(4,4,4-trifluorobutyryl)-5-phenyl-2-oxazolidinone (517 g) in dry tetrahydrofuran (800 mL) under an inert atmosphere. The mixture was maintained at -40 °C for one-half hour, and warmed to -35 °C over an additional one-half hour. To this mixture was added iodomethane (142 mL) over approximately 15 min while maintaining the internal reaction temperature between -35 °C and -30 °C. The mixture was stirred for an additional 2 h at -30 °C and the cold reaction mixture was poured over chilled aqueous ammonium chloride (700 g in 2 L water). The mixture was diluted with diethyl ether (1 L) and the layers separated. The organic layer was washed (25% w/v aqueous sodium bisulfate, brine). The aqueous portions were extracted with 1:1 methylene chloride:diethyl ether and methylene chloride. The combined organic layers were dried ($MgSO_4$) and evaporated to afford crude product (595 g) as a reddish oil. Filtration through silica gel (3000 mL), using a gradient of 1-5% ethyl acetate in hexanes, followed by evaporation, afforded a white solid (490 g) which was a mixture of the named product, the diastereomeric methylated side product and unmethylated starting material. Crystallization from diethyl ether:hexanes afforded (4R,5S)-4-methyl-3-((2R)-2-methyl-4,4,4-trifluorobutyryl)-5-phenyl-2-oxazolidinone (370 g, 68 %) as a white solid; mp 68-70°C. Analysis by HPLC (Zorbax silica gel, 4.6 mm x 25 cm, 1:9 ethyl acetate:hexanes, FR = 1.5 ml/min, UV detector at 254 nm) showed this sample to be about 99% pure (retention volume = 2.6). A second recrystallization of this white solid from diethyl ether:hexanes afforded an analytical sample of (4R,5S)-4-methyl-3((2R)-2-methyl-4,4,4-trifluorobutyryl)-5-phenyl-2-oxazolidinone (300 g, 55%) as transparent colorless needles; mp 74.5-75 °C; partial NMR (300 MHz, $CDCl_3$): 0.89 (d, 3H, 4-$CH_3$ of oxazolidinone), 1.33 (d, 3H, CH($CH_3$)CO), 2.10-2.31 (m, 1H, $CF_3CH_2$), 2.74-2.97 (m, 1H, $CF_3CH_2$), 4.03-4.17 (m, 1H, CHCO), 4.79 (m, 1H, 4-H of oxazolidinone), 5.71 (d, 1H, 5-H of oxazolidinone), 7.26-7.44 (m, 5H, phenyl). HPLC analysis as above showed 99.9% purity.

| Analysis for $C_{15}H_{16}F_3NO_3$: | | | |
|---|---|---|---|
| Calculated: | C, 57.14; | H, 5.11; | N, 4.44 |
| Found: | C, 57.17; | H, 5.16; | N, 4.59 |

e. (R)-2-Methyl-4,4,4-trifluorobutan-1-ol.

Lithium aluminum hydride (10.26 g) was added to a stirred solution of (4R,5S)-4-methyl-3-((2R)-2-methyl-4,4,4-trifluorobutyryl)-5-phenyl-2-oxazolidinone (28 g) in dry diethyl ether (200 mL) at -20 °C under an inert atmosphere, then the mixture was warmed to 0 °C. After 2 h at 0 °C, water (10.27 mL), 10% w/v sodium hydroxide (10.27 mL) and water (31 mL) were added, and the mixture was stirred 20 min. The salts were filtered and washed with distilled diethyl ether. The diethyl ether solution was dried ($K_2CO_3$) and diluted with pentane. This resulted in precipitation of recovered (4R,5S)-(+)-4-methyl-5-phenyl-2-oxazolidinone which was isolated by filtration. Concentration of the filtrate by distillation afforded several fractions. The first fractions (bath temperature to 60 °C) were pentane and diethyl ether; a second set of fractions (bath temperature 60 °C to 100 °C) was 12 g of a oil that was a 40:60 mixture of (R)-2-methyl-4,4,4-trifluorobutan-1-ol (calculated as 4.8 g alcohol) and diethyl ether by NMR. Warming the remaining tarry residue (bath temperature 85 °C) under vacuum (13,330 Pa) afforded an additional 7.2 g of (R)-2-methyl-4,4,4-trifluorobutan-1-ol (total yield, 12.0 g, 94%); partial NMR (300 MHz, $CDCl_3$-$D_2O$ shake): 1.06 (d, 3H, $CH_3$), 1.41 (br t, 1H, OH), 1.86-2.07 (m, 2H, CH($CH_3$) plus one $CF_3CH_2$), 2.31-2.42 (m, 1H, one $CF_3CH_2$), 3.49 (dd, 1H, one $CH_2OH$), 3.58 (dd, 1H, one $CH_2OH$).

f. (R)-2-(2-Methyl-4,4,4-trifluorobutyl)-1H-isoindol-1,3(2H)-dione.

Diethyl azodicarboxylate (15.4 mL) was added to a 0 °C, stirred slurry of (R)-2-methyl-4,4,4-trifluorobutan-1-ol (about 12.0 g), phthalimide (13.4 g), and triphenylphosphine (23.7 g) in diethyl ether (about 6.5 g, see above) and dry

tetrahydrofuran (110 mL), warmed to room temperature overnight, and stirred an additional 8 h. The mixture was evaporated, methylene chloride was added to the residue, and the slurry was filtered. The filtrate was purified by flash chromatography, eluting with 1:1 methylene chloride:hexanes, to give (R)-2-(2-methyl-4,4,4-trifluorobutyl)-1H-isoindol-1,3(2H)-dione (17.1 g, 75%) as a white solid; mp 45-47 °C; partial NMR (400 MHz, $CDCl_3$): 1.08 (d, 3H, $CH_3$), 1.94-2.07 (m, 1H, $CF_3C\underline{H}_2$), 2.14-2.31 (m, 1H, $CF_3C\underline{H}_2$), 2.36-2.50 (m, 1H, $C\underline{H}CH_3$), 3.58 (dd, 1H, $CH_2N$), 3.64 (dd, 1H, $CH_2N$).

g. (R)-2-Methyl-4,4,4-trifluorobutylamine hydrochloride.

Hydrazine monohydrate (3.1 mL) was added to a stirred solution of (R)-2-(2-methyl-4,4,4-trifluorobutyl)-1H-isoindole-1,3(2H)-dione (17.1 g) in anhydrous ethanol (85 mL) and heated to reflux. After three hours' reflux, the solution was cooled; ethanol (40 mL) was added; and the solution was acidifed to pH 1 by addition of concentrated hydrochloric acid and was filtered. The filtrate was evaporated, and the residue was purified by sublimation (bath temperature 170 °C, at 6.6 Pa) to yield (R)-2-methyl-4,4,4-trifluorobutylamine hydrochloride as a white solid (9.89 g, 88%); mp 187-191 °C; partial NMR (300 MHz, DMSO-$d_6$-$D_2O$ shake): 1.05 (d, 3H, $CH_3$), 2.06-2.36 (m, 2H, $CF_3C\underline{H}_2$) 2.36-2.54 (m, 1H, $C\underline{H}CH_3$) 2.73 (dd, 1H, $CH_2N$), 2.87 (dd, 1H, $CH_2N$) 8.20 (br s, 2H, $NH_2$).

h. Methyl (R)-3-methoxy-4-[1-methyl-5-(2-methyl-4,4,4-trifluorobutylcarbamoyl)indol-3-ylmethyl]benzoate.

A mixture of (R)-2-methyl-4,4,4-trifluorobutylamine hydrochloride (9.79 g), methyl 4-(5-carboxy-1-methylindol-3-ylmethyl)-3-methoxybenzoate (20.55 g), 4-dimethylaminopyridine (7.45 g), 1-(3-dimethylaminopropyl)-3-ethylcarbodiimide hydrochloride (15.07 g) and triethylamine (9.3 mL) was dissolved in dry methylene chloride (240 mL) and the solution was stirred under an inert atmosphere for 18 h. The mixture was diluted with methylene chloride (200 mL) and washed twice with 1N hydrochloric acid. The combined acid washes were back extracted with methylene chloride and the combined organic extracts were washed (water, brine), dried ($MgSO_4$), and evaporated. The residue was purified by flash chromatography, eluting with 97:3 methylene chloride:ethyl acetate to give methyl (R)-3-methoxy-4-[1-methyl-5-(2-methyl-4,4,4-trifluorobutylcarbamoyl)indol-3-ylmethyl]benzoate (14.48 g, 55%) as a white solid; mp 150-151 °C; partial NMR (300 MHz, $CDCl_3$): 1.12 (d, 3H, $CH_3$), 1.98-2.08 (m, 1H, $C\underline{H}CH_3$) 2.12-2.44 (m, 2H, $CF_3C\underline{H}_2$), 3.30-3.58 (m, 2H, $CH_2N$), 3.76 (s, 3H, $NCH_3$), 3.90 (s, 3H, $OCH_3$), 3.93 (s, 3H, $OCH_3$), 4.13 (s, 2H, $ArC\underline{H}_2Ar'$), 6.23 (br t, 1H, NHCO).

i. (R)-3-Methoxy-4-[1-methyl-5-(2-methyl-4,4,4-trifluorobutylcarbamoyl)indol-3-ylmethyl]benzoic acid.

A solution of lithium hydroxide monohydrate (7.68 g) in water (50 mL) was added to a stirred solution of methyl (R)-3-methoxy-4-[1-methyl-5-(2-methyl-4,4,4-trifluorobutylcarbamoyl)indol-3-ylmethyl]benzoate (14.38 g) in methanol (120 mL) and distilled tetrahydrofuran (120 mL) under an inert atmosphere. After 18 h the solvent was evaporated, the residue was dissolved in water (250 mL) and distilled tetrahydrofuran (23 mL), acidified to pH 1 by addition of concentrated hydrochloric acid, and diluted with water (150 mL). The precipitate was collected and washed with water to give (R)-3-methoxy-4-[1-methyl-5-(2-methyl-4,4,4-trifluorobutylcarbamoyl)indol-3-ylmethyl]benzoic acid (14.28 g, 100%); mp 218-223 °C; partial NMR (300 MHz, DMSO-$d_6$): 1.00 (d, 3H, $CH_3$), 2.04-2.28 (m, 2H, $CF_3C\underline{H}_2$), 2.32-2.44 (m, 1H, $C\underline{H}CH_3$), 3.21 (br t, 2H, $CH_2N$), 3.76 (s, 3H, $NCH_3$), 3.90 (s, 3H, $OCH_3$), 4.07 (s, 2H, $ArC\underline{H}_2Ar'$), 8.43 (br t, 1H, NHCO).

Example 3

(R)-3-Methoxy-4-[1-methyl-5-(2-methyl-4,4,4-trifluorobutylcarbamoyl)indol-3-ylmethyl]-N-(2-methylphenylsulfonyl)benzamide.

To a mixture of 4-(5-carboxy-1-methylindol-3-ylmethyl)-3-methoxy-N-(2-methylphenylsulfonyl)benzamide (103.5 g), 4-dimethylaminopyridine (112.4 g), and 1-(3-dimethylaminopropyl)-3-ethylcarbodiimide hydrochloride (51.8 g) in tetrahydrofuran (distilled from sodium benzophenone ketyl) (2.0 L), which had been stirred for 2 hours, was added (R)-2-methyl-4,4,4-trifluorobutylamine hydrochloride (42.6 g); and the reaction mixture was stirred overnight (about 18 hours, incomplete reaction) then heated to reflux for two hours (complete reaction). The cooled reaction mixture was diluted with ethyl acetate (2 L) washed with 1 N hydrochloric acid (twice) and brine, dried ($MgSO_4$) and evaporated. The residue (138.6 g) was combined with impure product from similar procedures (28.0 g) and purified by flash chromatography, eluting with methylene chloride:ethyl acetate (sequentially, 1:0, 9:1 and 3:1) to afford a solid which was triturated twice with ether to give the crude title compound (135.2 g) which was recrystallised from ethanol (1.2 L) and acetone (0.3 L) (concentrated by boiling to about 0.9 L and refrigerated) and dried under vacuum to provide the title compound (117.1 g, 65% recovery) as a white crystalline solid; mp 141.5-143.5 °C; NMR (300 MHz, DMSO-$d_6$): 1.01 (d, 3H, $CH_3$), 2.0-

2.2 (m, 2H, $CF_3C\underline{H}_2$), 2.3-2.5 (m, 1H, $C\underline{H}CH_3$), 2.61 (s, 3H, $ArC\underline{H}_3$), 3.23 (br t, 2H, $CH_2N$), 3.76 (s, 3H, $NCH_3$), 3.92 (s, 3H, $OCH_3$), 4.07 (s, $ArC\underline{H}_2Ar'$), 7.13 (s, 1H), 7.17 (d, 2H), 7.38-7.69 (m, 6H), 7.72 (d, 1H), 8.05 (d, 1H), 8.11 (s, 1H), 8.46 (br t, 1H, NHCO).

| Analysis for $C_{31}H_{32}F_3N_3O_5S$: | | | |
|---|---|---|---|
| Calculated: | C, 60.48; | H, 5.24; | N, 6.83 |
| Found: | C, 60.47; | H, 5.27; | N, 6.67 |

The starting material 5-carboxyindole derivative may be prepared as follows:

a. 4-(5-Methoxycarbonyl-1-methylindol-3-ylmethyl)-3-methoxybenzoic acid.

To a solution of methyl 4-(5-methoxycarbonyl-1-methylindol-3-ylmethyl)-3-methoxybenzoate (105.1 g) in tetrahydrofuran (1.4 L) was added methanol (450 mL) and deionized water (450 mL), followed by an equimolar amount of lithium hydroxide monohydrate (12.00 g). After the reaction mixture had stirred about 20 hours, it was acidified to pH 2 by addition of 6N hydrochloric acid (60 ml). Evaporation of the organic solvents resulted in the precipitation of a crude product (104.2 g) which was filtered and dried under vacuum before it was recrystallized by dissolving it in boiling tetrahydrofuran (600 mL), addition of toluene (about 1.2 L) and concentration to about one liter. Following cooling and stirring overnight, filtration, and drying under vacuum, a first crop is (71.1 g) was obtained. A second, similar recrystallization of this material from tetrahydrofuran (500 mL) and toluene (1 L) afforded 4-(5-methoxycarbonyl-1-methylindol-3-ylmethyl)-3-methoxybenzoic acid (58.3 g, 57.7%) as an off-white solid; NMR (300 MHz, DMSO-$d_6$): 3.78 (s, 3H, $NCH_3$), 3.83 (s, 3H, $CO_2CH_3$), 3.92 (s, 3H, $OCH_3$), 4.07 (s, $ArC\underline{H}_2Ar'$), 7.17 (d, 1H), 7.18 (s, 1H), 7.43-7.50 (m, 3H), 7.75 (dd, 1H), 8.19 (d, 1H); the same benzoic acid obtained by a similar procedure, but purified by flash chromatography, eluting with (methylene chloride:tetrahydrofuran:acetic acid (sequentially, 1:0:0, 1:9:0, and 0:400:1) followed by isolation and drying under vacuum of crystals formed on standing in methylene chloride:tetrahydrofuran fractions, had mp 228.0-229.5 °C. An additional amount of the benzoic acid (23.6 g, 23.3%), as well as recovered diester (11.5 g, 10.7%), was obtained by concentration and flash chromatography of the mother liquors, eluting with methylene chloride:tetrahydrofuran (sequentially, 1:0, 3:1, 2:1).

b. 4-(5-Methoxycarbonyl-1-methylindol-3-ylmethyl)-3-methoxy-N-(2-methylphenylsulfonyl)benzamide.

To a solution of 4-(5-methoxycarbonyl-1-methylindol-3-ylmethyl)-3-methoxybenzoic acid (125.9 g) in tetrahydrofuran (3.0 L, distilled from sodium benzophenone ketyl) (prepared by heating at 50 °C until dissolution was complete, followed by cooling to room temperature with an ice-water bath) was added 4-dimethylaminopyridine (56.6 g) and 1-(3-dimethylaminopropyl)-3-ethylcarbodiimide hydrochloride (102.4 g), and the mixture was stirred one hour. To the mixture was added 2-methylbenzenesulfonamide (67.1 g), and the reaction mixture was stirred about 3 days (for convenience). The reaction mixture was diluted with ethyl acetate (2.0 L) and washed with 1N hydrochloric acid (twice) and brine (3 times, until neutral), and the aqueous extracts were back washed with ethyl acetate. The combined ethyl acetate solution was dried ($MgSO_4$), and partially evaporated to give a slurry of solid in ethyl acetate (about 0.5 L) which was refrigerated overnight. Collection of the solid afforded the crude product (158.5 g, 88%, essentially pure by TLC) as a light pink solid. Recrystallization by dissolution in hot tetrahydrofuran (1.5 L), filtration while hot, dilution with ethyl acetate (2.0 L), and boiling down to a final volume of about 2.5 L afforded a first crop of 4-(5-methoxycarbonyl-1-methylindol-3-ylmethyl)-3-methoxy-N-(2-methylphenylsulfonyl)benzamide (105.5 g, 59%) as a white solid; mp 211-213 °C; NMR (250 MHz, DMSO-$d_6$): 2.60 (s, 3H, $ArC\underline{H}_3$), 3.76 (s, 3H, $NCH_3$), 3.82 (s, 3H, $CO_2CH_3$), 3.92 (s, 3H, $ArOC\underline{H}_3$), 4.04 (s, 2H, $ArC\underline{H}_2Ar'$), 7.15 (d, 1H), 7.22 (s, 1H), 7.38-7.58 (m, 6H), 7.75 (dd, 1H), 8.03 (dd, 1H), 8.17 (d, 1H). (Two additional crops (35.5 g, 20%) and crude product (39.5 g) from concentration of the mother liquors were also obtained.)

c. 4-(5-Carboxy-1-methylindol-3-ylmethyl)-3-methoxy-N-(2-methylphenylsulfonyl)benzamide.

A mixture of 4-(5-methoxycarbonyl-1-methylindol-3-ylmethyl)-3-methoxy-N-(2-methylphenylsulfonyl)benzamide (130.0 g), tetrahydrofuran (1.0 L) and 1 N sodium hydroxide (1.0 L) was heated to about 60 °C overnight, then treated with additional 1N sodium hydroxide (200 mL) and heated an additional 5 hours at 60 °C (likely unnecessary). The cooled reaction mixture was acidified with 6 N hydrochloric acid (250 mL) and extracted with ethyl acetate. The ethyl acetate solution was washed with brine (three times), dried ($MgSO_4$) and evaporated to give a solid which was dried at 50 °C under vacuum to give 4-(5-carboxy-1-methylindol-3-ylmethyl)-3-methoxy-N-(2-methylphenylsulfonyl)benzamide

(12.9 g, 100% when calculated as 0.45 hydrate), mp 255-257 °C; NMR (300 MHz, DMSO-$d_6$): 2.60 (s, 3H, ArC$\underline{H}_3$), 3.76 (s, 3H, $NCH_3$), 3.91 (s, 3H, $OCH_3$), 4.05 (s, 2H, ArC$\underline{H}_2$Ar'), 7.15 (d, 1H), 7.19 (s, 1H), 7.39-7.51 (m, 5H), 7.58 (br t, 1H), 7.72 (dd, 1H), 8.03 (dd, 1H), 8.14 (d, 1H).

| Anaylsis for $C_{26}H_{24}N_2O_6S$.0.45 $H_2O$: | | | |
|---|---|---|---|
| Calculated: | C, 62.37; | H, 5.01; | N, 5.60 |
| Found: | C, 62.60; | H, 5.03; | N, 5.52 |

Methyl 4-(5-methoxycarbonyl-1-methylindol-3-ylmethyl)-3-methoxybenzoate, used in step a., above, may be obtained from methyl indole-5-carboxylate and methyl 4-bromomethyl-3-methoxybenzoate using a similar procedure to that described above in Example 1.b, followed by methylation using a similar procedure to that described above in Example 1.c. Methyl 4-(5-methoxycarbonyl-1-methylindol-3-ylmethyl)-3-methoxybenzoate, obtained by esterification of 4-(5-carboxy-1-methylindol-3-ylmethyl)-3-methoxybenzoic acid with methanol and acetyl chloride, followed by filtration through a bed of silica gel, eluting with methylene chloride in a continuous extractor, evaporation and trituration with ether, had mp 138-139 °C.

Example 4

The following illustrates representative pharmaceutical dosages forms which may be used for the therapeutic or prophylactic administration of a compound of formula I or of a pharmaceutically acceptable salt thereof (hereinafter referred to as 'Compound X'):

(i)

| Tablet 1 | mg/tablet |
|---|---|
| 'Compound X' | 100.0 |
| Lactose | 77.5 |
| Povidone | 15.0 |
| Croscarmellose sodium | 12.0 |
| Microcrystalline cellulose | 92.5 |
| Magnesium stearate | 3.0 |
| | 300.0 |

(ii)

| Tablet 2 | mg/tablet |
|---|---|
| 'Compound X' | 20.0 |
| Microcrystalline cellulose | 410.0 |
| Starch | 50.0 |
| Sodium starch glycolate | 15.0 |
| Magnesium stearate | 5.0 |
| | 500.0 |

(iii)

| Capsule | mg/capsule |
|---|---|
| 'Compound X' | 10.0 |
| Colloidal silicon dioxide | 1.5 |
| Lactose | 465.5 |
| Pregelatinized starch | 120.0 |
| Magnesium stearate | 3.0 |
| | 600.0 |

(iv)

| Injection 1 (1 mg/mL) | mg/mL |
|---|---|
| 'Compound X' (free acid form) | 1.0 |
| Dibasic sodium phosphate | 12.0 |
| Monobasic sodium phosphate | 0.7 |
| Sodium chloride | 4.5 |
| 1.0 N Sodium hydroxide solution (pH adjustment to 7.0-7.5) | q.s. |
| Water for injection | q.s. ad 1 mL |

(v)

| Injection 2 (10 mg/mL) | mg/mL |
|---|---|
| 'Compound X' (free acid form) | 10.0 |
| Monobasic sodium phosphate | 0.3 |
| Dibasic sodium phosphate | 1.1 |
| Polyethylene glycol 400 | 200.0 |
| 0.1 N Sodium hydroxide solution (pH adjustment to 7.0-7.5) | q.s. |
| Water for injection | q.s. ad 1 mL |

(vi)

| Aerosol | mg/can |
|---|---|
| 'Compound X' | 20.0 |
| Oleic acid | 10.0 |
| Trichloromonofluoromethane | 5,000.0 |
| Dichlorodifluoromethane | 10,000.0 |
| Dichlorotetrafluoroethane | 5,000.0 |

It will be appreciated that the above pharmaceutical compositions may be varied according to well-known pharmaceutical techniques to accomodate differing amounts and types of active ingredient 'Compound X'. The aerosol (vi) may

be used in conjunction with a standard, metered dose aerosol dispenser.

Formulae

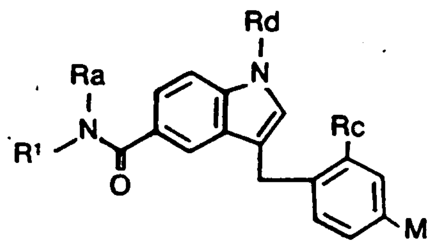

Ia

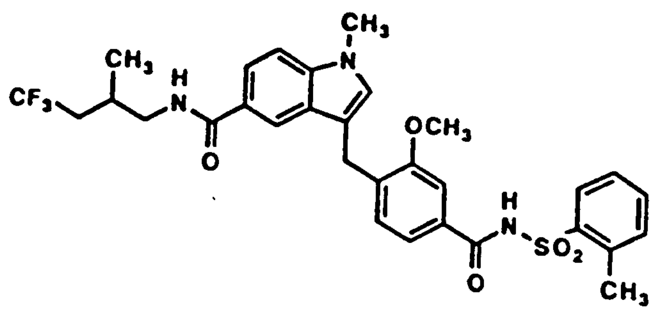

I

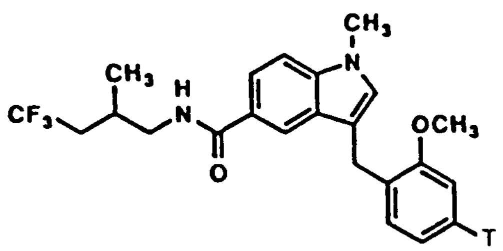

II

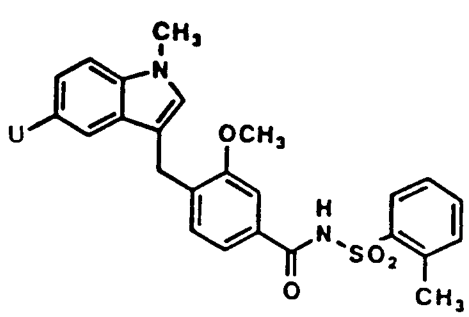

III

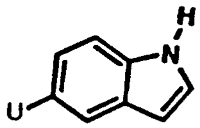

IV

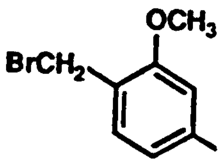

V

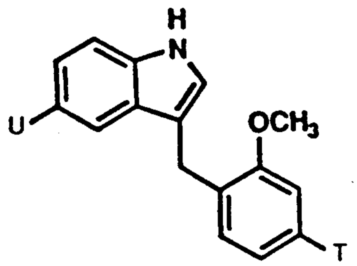

VI

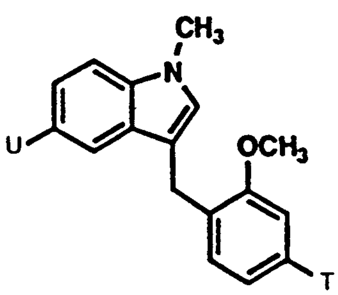

VII

## Claims

**Claims for the following Contracting States : AT, BE, CH, DE, DK, FR, GB, IT, LI, LU, NL, SE**

1. 3-Methoxy-4-[1-methyl-5-(2-methyl-4,4,4-trifluorobutylcarbamoyl)indol-3-ylmethyl]-N-(2-methylphenylsulfonyl)benzamide, or a pharmaceutically acceptable salt thereof.

2. A compound as claimed in Claim 1 which is in racemic form.

3. A compound as claimed in Claim 1 which is of the substantially pure (R)-form.

4. A pharmaceutically acceptable salt as claimed in any of Claims 1-3 which is a lithium, sodium or potassium salt.

5. 3-Methoxy-4-[1-methyl-5-(2-methyl-4,4,4-trifluorobutylcarbamoyl)indol-3-ylmethyl]-N-(2-methylphenylsulfonyl)benzamide which is of the substantially pure (R)-form.

6. A method of making a compound as claimed in any one of Claims 1-5 which is characterized by:

(A) reacting a corresponding 3-methoxy-4-[1-methyl-5-(2-methyl-4,4,4-trifluorobutylcarbamoyl)indol-3-ylmethyl]benzoic acid with 2-methylbenzenesulfonamide in the presence of a dehydrating agent or reacting a reactive derivative of said benzoic acid with 2-methylbenzenesulfonamide, or a salt thereof; or
(B) acylating 2-methyl-4,4,4-trifluorobutylamine with 4-(5-carboxy-1-methylindol-3-ylmethyl)-3-methoxy-N-(2-methylphenylsulfonyl)benzamide in the presence of a dehydrating agent or with a reactive derivative of said carboxyindole;

wherein, when a compound in substantially enantiomerically pure form is required, optionally performing said method using a substantially enantiomerically pure starting material;

whereafter, alternatively, when a compound in substantially enantiomerically pure form is required; separating the desired optically active form using a conventional method;

and whereafter, when a pharmaceutically acceptable salt is required, reacting the 3-methoxy-4-[1-methyl-5-(2-methyl-4,4,4-trifluorobutylcarbamoyl)indol-3-ylmethyl]-N-(2-methylphenylsulfonyl)benzamide with a suitable base which affords a physiologically acceptable cation.

7. A pharmaceutical composition which comprises a compound as claimed in any one of Claims 1-5 together with a pharmaceutically acceptable diluent or carrier.

8. 3-Methoxy-4-[1-methyl-5-(2-methyl-4,4,4-trifluorobutylcarbamoyl)indol-3-ylmethyl]benzoic acid, or an ester of said benzoic acid with an alcohol of formula $HOR^h$ wherein $R^h$ is a conveniently removed acid protecting group, or a base addition salt of said benzoic acid.

9. 2-Methyl-4,4,4-trifluorobutylamine, or an acid addition salt thereof.

10. A compound as claimed in Claim 9 which is of the substantially pure (R)-form.

**Claims for the following Contracting State : ES**

1. A process for producing 3-methoxy-4-[1-methyl-5-(2-methyl-4,4,4-trifluorobutylcarbamoyl)indol-3-ylmethyl]-N-(2-methylphenylsulfonyl)benzamide, or a pharmaceutically acceptable salt thereof, which method is selected from

(A) reacting a corresponding 3-methoxy-4-[1-methyl-5-(2-methyl-4,4,4-trifluorobutylcarbamoyl)indol-3-ylmethyl]benzoic acid with 2-methylbenzenesulfonamide in the presence of a dehydrating agent or reacting a reactive derivative of said benzoic acid with 2-methylbenzenesulfonamide, or a salt thereof; and
(B) acylating 2-methyl-4,4,4-trifluorobutylamine with 4-(5-carboxy-1-methylindol-3-ylmethyl)-3-methoxy-N-(2-methylphenylsulfonyl)benzamide in the presence of a dehydrating agent or with a reactive derivative of said carboxyindole;

wherein, when a compound in substantially enantiomerically pure form is required, optionally performing said method using a substantially enantiomerically pure starting material;

whereafter, alternatively, when a compound in substantially enantiomerically pure form is required; separating the desired optically active form using a conventional method;

and whereafter, when a pharmaceutically acceptable salt is required, reacting the 3-methoxy-4-[1-methyl-5-(2-methyl-4,4,4-trifluorobutylcarbamoyl)indol-3-ylmethyl]-N-(2-methylphenylsulfonyl)benzamide with a suitable base which affords a physiologically acceptable cation.

2. 3-Methoxy-4-[1-methyl-5-(2-methyl-4,4,4-trifluorobutylcarbamoyl)indol-3-ylmethyl]-N-(2-methylphenylsulfonyl)benzamide, or a pharmaceutically acceptable salt thereof.

3. A compound as claimed in Claim 2 which is in racemic form.

4. A compound as claimed in Claim 2 which is of the substantially pure (R)-form.

5. 3-Methoxy-4-[1-methyl-5-(2-methyl-4,4,4-trifluorobutylcarbamoyl)indol-3-ylmethyl]-N-(2-methylphenylsulfonyl)benzamide which is of the substantially pure (R)-form.

**Claims for the following Contracting State : GR**

1. A process for producing 3-methoxy-4-[1-methyl-5-(2-methyl-4,4,4-trifluorobutylcarbamoyl)indol-3-ylmethyl]-N-(2-methylphenylsulfonyl)benzamide, or a pharmaceutically acceptable salt thereof, which method is selected from

   (A) reacting a corresponding 3-methoxy-4-[1-methyl-5-(2-methyl-4,4,4-trifluorobutylcarbamoyl)indol-3-ylmethyl]benzoic acid with 2-methylbenzenesulfonamide in the presence of a dehydrating agent or reacting a reactive derivative of said benzoic acid with 2-methylbenzenesulfonamide, or a salt thereof; and
   (B) acylating 2-methyl-4,4,4-trifluorobutylamine with 4-(5-carboxy-1-methylindol-3-ylmethyl)-3-methoxy-N-(2-methylphenylsulfonyl)benzamide in the presence of a dehydrating agent or with a reactive derivative of said carboxyindole;

   wherein, when a compound in substantially enantiomerically pure form is required, optionally performing said method using a substantially enantiomerically pure starting material;

   whereafter, alternatively, when a compound in substantially enantiomerically pure form is required; separating the desired optically active form using a conventional method;

   and whereafter, when a pharmaceutically acceptable salt is required, reacting the 3-methoxy-4-[1-methyl-5-(2-methyl-4,4,4-trifluorobutylcarbamoyl)indol-3-ylmethyl]-N-(2-methylphenylsulfonyl)benzamide with a suitable base which affords a physiologically acceptable cation.

2. 3-Methoxy-4-[1-methyl-5-(2-methyl-4,4,4-trifluorobutylcarbamoyl)indol-3-ylmethyl]benzoic acid, or an ester of said benzoic acid with an alcohol of formula $HOR^h$ wherein $R^h$ is a conveniently removed acid protecting group, or a base addition salt of said benzoic acid.

3. 2-Methyl-4,4,4-trifluorobutylamine, or an acid addition salt thereof.

4. 3-Methoxy-4-[1-methyl-5-(2-methyl-4,4,4-trifluorobutylcarbamoyl)indol-3-ylmethyl]-N-(2-methylphenylsulfonyl)benzamide, or a pharmaceutically acceptable salt thereof.

5. A compound as claimed in Claim 4 which is in racemic form.

6. A compound as claimed in Claim 4 which is of the substantially pure (R)-form.

7. 3-Methoxy-4-[1-methyl-5-(2-methyl-4,4,4-trifluorobutylcarbamoyl)indol-3-ylmethyl]-N-(2-methylphenylsulfonyl)benzamide which is of the substantially pure (R)-form.

**Patentansprüche**

**Patentansprüche für folgende Vertragsstaaten : AT, BE, CH, DE, DK, FR, GB, IT, LI, LU, NL, SE**

1. 3-Methoxy-4-[1-methyl-5-(2-methyl-4,4,4-trifluorbutylcarbamoyl)indol-3-ylmethyl]-N-(2-methylphenylsulfonyl)benzamid oder ein pharmazeutisch akzeptables Salz davon.

2. Verbindung nach Anspruch 1 in racemischer Form.

3. Verbindung nach Anspruch 1 in der im wesentlichen reinen (R)-Form.

4. Pharmazeutisch akzeptables Salz nach einem der Ansprüche 1 bis 3, bei dem es sich um ein Lithium-, Natrium- oder Kaliumsalz handelt.

5. 3-Methoxy-4-[1-methyl-5-(2-methyl-4,4,4-trifluorbutylcarbamoyl)indol-3-ylmethyl]-N-(2-methylphenylsulfonyl)benzamid in der im wesentlichen reinen (R)-Form.

6. Verfahren zur Herstellung einer Verbindung nach einem der Ansprüche 1 bis 5, das

dadurch **gekennzeichnet** ist, daß:

(A) eine entsprechende 3-Methoxy-4-[1-methyl-5-(2-methyl-4,4,4-trifluorbutylcarbamoyl)indol-3-ylmethyl]benzoesäure mit 2-Methylbenzolsulfonamid in Gegenwart eines Dehydratisierungsmittels umgesetzt wird oder ein reaktives Derivat der Benzoesäure mit 2-Methylbenzolsulfonamid, oder einem Salz davon, umgesetzt wird, oder

(B) 2-Methyl-4,4,4-trifluorbutylamin mit 4-(5-Carboxy-1-methylindol-3-ylmethyl)-3-methoxy-N-(2-methylphenylsulfonyl)benzamid in Gegenwart eines Dehydratisierungsmittels oder mit einem reaktiven Derivat des Carboxyindols acyliert wird,
wobei, wenn eine Verbindung in im wesentlichen enantiomerenreiner Form erforderlich ist, das Verfahren gegebenenfalls unter Verwendung eines im wesentlichen enantiomerenreinen Ausgangsmaterials durchgeführt wird,
worauf alternativ, wenn eine Verbindung in im wesentlichen enantiomerenreiner Form erforderlich ist, die gewünschte optisch aktive Form unter Anwendung eines herkömmlichen Verfahrens abgetrennt wird,
und worauf, wenn ein pharmazeutisch akzeptables Salz erforderlich ist, das 3-Methoxy-4-[1-methyl-5-(2-methyl-4,4,4-trifluorbutylcarbamoyl)indol-3-ylmethyl]-N-(2-methylphenylsulfonyl)benzamid mit einer geeigneten Base, die ein physiologisch akzeptables Kation liefert, umgesetzt wird.

**7.** Pharmazeutische Zusammensetzung, die eine Verbindung nach einem der Ansprüche 1 bis 5 in Verbindung mit einem pharmazeutisch akzeptablen Verdünnungsmittel oder Trägermittel enthält.

**8.** 3-Methoxy-4-[1-methyl-5-(2-methyl-4,4,4-trifluorbutylcarbamoyl)indol-3-ylmethyl]-benzoesäure, oder ein Ester dieser Benzoesäure mit einem Alkohol der Formel $HOR^h$, wobei $R^h$ eine bequem zu entfernende Säure-Schutzgruppe ist, oder ein Basenadditionssalz dieser Benzoesäure.

**9.** 2-Methyl-4,4,4-trifluorbutylamin oder ein Säureadditionssalz davon.

**10.** Verbindung nach Anspruch 9 in der im wesentlichen reinen (R)-Form.

**Patentansprüche für folgenden Vertragsstaat : ES**

**1.** Verfahren zur Herstellung von 3-Methoxy-4-[1-methyl-5-(2-methyl-4,4,4-trifluorbutylcarbamoyl)indol-3-ylmethyl]-N-(2-methylphenylsulfonyl)benzamid oder eines pharmazeutisch akzeptablen Salzes davon, wobei das Verfahren aus Verfahren ausgewählt ist, bei denen:

(A) eine entsprechende 3-Methoxy-4-[1-methyl-5-(2-methyl-4,4,4-trifluorbutylcarbamoyl)indol-3-ylmethyl]benzoesäure mit 2-Methylbenzolsulfonamid in Gegenwart eines Dehydratisierungsmittels umgesetzt wird oder ein reaktives Derivat der Benzoesäure mit 2-Methylbenzolsulfonamid, oder einem Salz davon, umgesetzt wird, und

(B) 2-Methyl-4,4,4-trifluorbutylamin mit 4-(5-Carboxy-1-methylindol-3-ylmethyl)-3-methoxy-N-(2-methylphenylsulfonyl)benzamid in Gegenwart eines Dehydratisierungsmittels oder mit einem reaktiven Derivat des Carboxyindols acyliert wird,
wobei, wenn eine Verbindung in im wesentlichen enantiomerenreiner Form erforderlich ist, das Verfahren gegebenenfalls unter Verwendung eines im wesentlichen enantiomerenreinen Ausgangsmaterials durchgeführt wird,
worauf alternativ, wenn eine Verbindung in im wesentlichen enantiomerenreiner Form erforderlich ist, die gewünschte optisch aktive Form unter Anwendung eines herkömmlichen Verfahrens abgetrennt wird,
und worauf, wenn ein pharmazeutisch akzeptables Salz erforderlich ist, das 3-Methoxy-4-[1-methyl-5-(2-methyl-4,4,4-trifluorbutylcarbamoyl)indol-3-ylmethyl]-N-(2-methylphenylsulfonyl)benzamid mit einer geeigneten Base, die ein physiologisch akzeptables Kation liefert, umgesetzt wird.

**2.** 3-Methoxy-4-[1-methyl-5-(2-methyl-4,4,4-trifluorbutylcarbamoyl)indol-3-ylmethyl]-N-(2-methylphenylsulfonyl)benzamid oder ein pharmazeutisch akzeptables Salz davon.

**3.** Verbindung nach Anspruch 2 in racemischer Form.

4. Verbindung nach Anspruch 2 in der im wesentlichen reinen (R)-Form.

5. 3-Methoxy-4-[1-methyl-5-(2-methyl-4,4,4-trifluorbutylcarbamoyl)indol-3-ylmethyl]-N-(2-methylphenylsulfonyl)benzamid in der im wesentlichen reinen (R)-Form.

**Patentansprüche für folgenden Vertragsstaat : GR**

1. Verfahren zur Herstellung von 3-Methoxy-4-[1-methyl-5-(2-methyl-4,4,4-trifluorbutylcarbamoyl)indol-3-ylmethyl]-N-(2-methylphenylsulfonyl)benzamid oder eines pharmazeutisch akzeptablen Salzes davon, wobei das Verfahren aus Verfahren ausgewählt ist, bei denen:

   (A) eine entsprechende 3-Methoxy-4-[1-methyl-5-(2-methyl-4,4,4-trifluorbutylcarbamoyl)indol-3-ylmethyl)benzoesäure mit 2-Methylbenzolsulfonamid in Gegenwart eines Dehydratisierungsmittels umgesetzt wird oder ein reaktives Derivat der Benzoesäure mit 2-Methylbenzolsulfonamid, oder einem Salz davon, umgesetzt wird, und

   (B) 2-Methyl-4,4,4-trifluorbutylamin mit 4-(5-Carboxy-1-methylindol-3-ylmethyl)-3-methoxy-N-(2-methylphenylsulfonyl)benzamid in Gegenwart eines Dehydratisierungsmittels oder mit einem reaktiven Derivat des Carboxyindols acyliert wird,
   wobei, wenn eine Verbindung in im wesentlichen enantiomerenreiner Form erforderlich ist, das Verfahren gegebenenfalls unter Verwendung eines im wesentlichen enantiomerenreinen Ausgangsmaterials durchgeführt wird,
   worauf alternativ, wenn eine Verbindung in im wesentlichen enantiomerenreiner Form erforderlich ist, die gewünschte optisch aktive Form unter Anwendung eines herkömmlichen Verfahrens abgetrennt wird,
   und worauf, wenn ein pharmazeutisch akzeptables Salz erforderlich ist, das 3-Methoxy-4-[1-methyl-5-(2-methyl-4,4,4-trifluorbutylcarbamoyl)indol-3-ylmethyl]-N-(2-methylphenylsulfonyl)benzamid mit einer geeigneten Base, die ein physiologisch akzeptables Kation liefert, umgesetzt wird.

2. 3-Methoxy-4-[1-methyl-5-(2-methyl-4,4,4-trifluorbutylcarbamoyl)indol-3-ylmethyl]benzoesäure, oder ein Ester dieser Benzoesäure mit einem Alkohol der Formel $HOR^h$, wobei $R^h$ eine leicht zu entfernende Säure-Schutzgruppe ist, oder ein Basenadditionssalz dieser Benzoesäure.

3. 2-Methyl-4,4,4-trifluorbutylamin oder ein Säureadditionssalz davon.

4. 3-Methoxy-4-[1-methyl-5-(2-methyl-4,4,4-trifluorbutylcarbamoyl)indol-3-ylmethyl]-N-(2-methylphenylsulfonyl)benzamid oder ein pharmazeutisch akzeptables Salz davon.

5. Verbindung nach Anspruch 4 in racemischer Form.

6. Verbindung nach Anspruch 4 in der im wesentlichen reinen (R)-Form.

7. 3-Methoxy-4-[1-methyl-5-(2-methyl-4,4,4-trifluorbutylcarbamoyl)indol-3-ylmethyl]-N-(2-methylphenylsulfonyl)benzamid in der im wesentlichen reinen (R)-Form.

**Revendications**

**Revendications pour les Etats contractants suivants : AT, BE, CH, DE, DK, FR, GB, IT, LI, LU, NL, SE**

1. 3-méthoxy-4-[1-méthyl-5-(2-méthyl-4,4,4-trifluorobutylcarbamoyl)indole-3-ylméthyl]-N-(2-méthylphénylsulfonyl)benzamide, ou un de ses sels pharmaceutiquement acceptables.

2. Composé suivant la revendication 1, qui est sous forme racémique.

3. Composé suivant la revendication 1, qui est constitué de la forme (R) pratiquement pure.

4. Sel pharmaceutiquement acceptable d'un composé suivant l'une quelconque des revendications 1 à 3, qui est un sel de lithium, de sodium ou de potassium.

**5.** 3-méthoxy-4-[1-méthyl-5-(2-méthyl-4,4,4-trifluorobutylcarbamoyl)indole-3-ylméthyl]-N-(2-méthylphénylsulfonyl)benzamide, qui est sous la forme (R) pratiquement pure.

**6.** Procédé de préparation d'un composé suivant l'une quelconque des revendications 1 à 5, qui est caractérisé par :

(A) la réaction d'un acide 3-méthoxy-4-[1-méthyl-5-(2-méthyl-4,4,4-trifluorobutylcarbamoyl)indole-3-ylméthyl]benzoïque correspondant avec le 2-méthylbenzènesulfonamide en présence d'un agent déshydratant, ou la réaction d'un dérivé réactif de cet acide benzoïque avec le 2-méthylbenzènesulfonamide, ou un de ses sels ; ou
(B) l'acylation de 2-méthyl-4,4,4-trifluorobutylamine avec le 4-(5-carboxy-1-méthylindole-3-ylméthyl)-3-méthoxy-N-(2-méthylphénylsulfonyl)benzamide en présence d'un agent déshydratant ou avec un dérivé réactif de ce carboxyindole ;

ledit procédé, lorsqu'un composé sous une forme pratiquement énantiomériquement pure est requis, étant facultativement mis en oeuvre en utilisant une matière de départ pratiquement énantiomériquement pure ;

puis, en variante, lorsqu'un composé sous une forme pratiquement énantiomériquement pure est requis, la séparation de la forme optiquement active désirée en utilisant un procédé classique ;

et ensuite, lorsqu'un sel pharmaceutiquement acceptable est requis, la réaction du 3-méthoxy-4-[1-méthyl-5-(2-méthyl-4,4,4-trifluorobutylcarbamoyl)indole-3-ylméthyl]-N-(2-méthylphénylsulfonyl)benzamide avec une base convenable qui donne un cation physiologiquement acceptable.

**7.** Composition pharmaceutique qui comprend un composé suivant l'une quelconque des revendications 1 à 5, en association avec un diluant ou support pharmaceutiquement acceptable.

**8.** Acide 3-méthoxy-4-[1-méthyl-5-(2-méthyl-4,4,4-trifluorobutylcarbamoyl)indole-3-ylméthyl]-benzoïque ou un ester de cet acide benzoïque avec un alcool de formule $HOR^h$ dans laquelle $R^h$ représente un groupe protecteur de la fonction acide pouvant être éliminé convenablement, ou un sel d'addition de base de cet acide benzoïque.

**9.** 2-méthyl-4,4,4-trifluorobutylamine, ou un de ses sels d'addition d'acides.

**10.** Composé suivant la revendication 9, qui est sous la forme (R) pratiquement pure.

**Revendications pour l'Etat contractant suivant : ES**

**1.** Procédé pour la production de 3-méthoxy-4-[1-méthyl-5-(2-méthyl-4,4,4-trifluorobutylcarbamoyl)indole-3-ylméthyl]-N-(2-méthylphénylsulfonyl)benzamide, ou d'un de ses sels pharmaceutiquement acceptables, procédé qui est choisi entre

(A) la réaction d'un acide 3-méthoxy-4-[1-méthyl-5-(2-méthyl-4,4,4-trifluorobutylcarbamoyl)indole-3-ylméthyl]benzoïque correspondant avec le 2-méthylbenzènesulfonamide en présence d'un agent déshydratant, ou la réaction d'un dérivé réactif de cet acide benzoïque avec le 2-méthylbenzènesulfonamide, ou un de ses sels ; et
(B) l'acylation de la 2-méthyl-4,4,4-trifluorobutylamine avec le 4-(5-carboxy-1-méthylindole-3-ylméthyl)-3-méthoxy-N-(2-méthylphénylsulfonyl)benzamide en présence d'un agent déshydratant ou avec un dérivé réactif de ce carboxyindole ;

ledit procédé, lorsqu'un composé sous une forme pratiquement énantiomériquement pur est requis, étant facultativement mis en oeuvre en utilisant une matière de départ pratiquement énantiomériquement pure ;

puis, en variante, lorsqu'un composé sous une forme pratiquement énantiomériquement pure est requis, la séparation de la forme optiquement active désirée en utilisant un procédé classique ;

et ensuite, lorsqu'un sel pharmaceutiquement acceptable est requis, la réaction du 3-méthoxy-4-[1-méthyl-5-(2-méthyl-4,4,4-trifluorobutylcarbamoyl)indole-3-ylméthyl]-N-(2-méthylphénylsulfonyl)benzamide avec une base convenable qui donne un cation physiologiquement acceptable.

**2.** 3-méthoxy-4-[1-méthyl-5-(2-méthyl-4,4,4-trifluorobutylcarbamoyl)indole-3-ylméthyl]-N-(2-méthylphénylsulfonyl)benzamide, ou un se ses sels pharmaceutiquement acceptables.

**3.** Composé suivant la revendication 2, qui est sous forme racémique.

4. Composé suivant la revendication 2, qui est sous la forme (R) pratiquement pure.

5. 3-méthoxy-4-[1-méthyl-5-(2-méthyl-4,4,4-trifluorobutylcarbamoyl)indole-3-ylméthyl]-N-(2-méthylphénylsulfonyl)benzamide, qui est sous la forme (R) pratiquement pure.

**Revendications pour l'Etat contractant suivant : GR**

1. Procédé pour la production de 3-méthoxy-4-[1-méthyl-5-(2-méthyl-4,4,4-trifluorobutylcarbamoyl)indole-3-ylméthyl]-N-(2-méthylphénylsulfonyl)benzamide, ou d'un des ses sels pharmaceutiquement acceptables, procédé qui est choisi entre

   (A) la réaction d'un acide 3-méthoxy-4-[1-méthyl-5-(2-méthyl-4,4,4-trifluorobutylcarbamoyl)indole-3-ylméthyl]benzoïque correspondant avec le 2-méthylbenzènesulfonamide en présence d'un agent déshydratant, ou la réaction d'un dérivé réactif de cet acide benzoïque avec le 2-méthylbenzènesulfonamide, ou un de ses sels ; et
   (B) l'acylation de la 2-méthyl-4,4,4-trifluorobutylamine avec le 4-(5-carboxy-1-méthylindole-3-ylméthyl)-3-méthoxy-N-(2-méthylphénylsulfonyl)benzamide en présence d'un agent déshydratant ou avec un dérivé réactif de ce carboxyindole ;

   ledit procédé, lorsqu'un composé sous une forme pratiquement énantiomériquement pure est requis, étant facultativement mis en oeuvre en utilisant une matière de départ pratiquement énantiomériquement pure ;

   puis, en variante, lorsqu'un composé sous une forme pratiquement énantiomériquement pure est requis, la séparation de la forme optiquement active désirée en utilisant un procédé classique ;

   et ensuite, lorsqu'un sel pharmaceutiquement acceptable est requis, la réaction du 3-méthoxy-4-[1-méthyl-5-(2-méthyl-4,4,4-trifluorobutylcarbamoyl)indole-3-ylméthyl]-N-(2-méthylphénylsulfonyl)benzamide avec une base convenable qui donne un cation physiologiquement acceptable.

2. Acide 3-méthoxy-4-[1-méthyl-5-(2-méthyl-4,4,4-trifluorobutylcarbamoyl)indole-3-ylméthyl]-benzoïque, ou un ester de cet acide benzoïque avec un alcool de formule $HOR^h$ dans laquelle $R^h$ représente un groupe protecteur de la fonction acide pouvant être éliminé convenablement, ou un sel d'addition de base de cet acide benzoïque.

3. 2-méthyl-4,4,4-trifluorobutylamine ou un de ses sels d'additions d'acides.

4. 3-méthoxy-4-[1-méthyl-5-(2-méthyl-4,4,4-trifluorobutylcarbamoyl)indole-3-ylméthyl]-N-(2-méthylphénylsulfonyl)benzamide, ou un de sels pharmaceutiquement acceptables.

5. Composé suivant la revendication 4, qui est sous forme racémique.

6. Composé suivant la revendication 4, qui est sous la forme (R) pratiquement pure.

7. 3-méthoxy-4-[1-méthyl-5-(2-méthyl-4,4,4-trifluorobutylcarbamoyl)indole-3-ylméthyl]-N-(2-méthylphénylsulfonyl)benzamide, qui est sous la forme (R) pratiquement pure.